# EUROPEAN PATENT SPECIFICATION

(11) **EP 0 404 967 B1**
(45) Date of publication and mention of the grant of the patent: **02.11.1995**
(21) Application number: 90901894.7
(22) Date of filing: 17.01.1990
(51) Int. Cl.: C12N 15/53, C12N 9/80

(54) **RECOMBINANT C-TERMINAL ALPHA-AMIDATING ENZYME OF HUMAN THYROID ORIGIN**
REKOMBINANTES C-TERMINAL ALPHA AMIDATIERUNGSENZYM VON MENSCHLISCHEM THYROIDURSPRUNG
ENZYME RECOMBINANTE POUR L'ALPHA AMIDATION DU C TERMINAL, PROVENANT DE LA THYROIDE HUMAINE

(30) Priority: 17.01.1989 JP 5878/89
(43) Date of publication of application: 02.01.1991
(73) Proprietor: SUNTORY LIMITED, Kita-ku, Osaka-shi, Osaka 530 (JP)
(72) Inventor: OHSUYE, Kazuhiro, Osaka 567 (JP); KITANO, Katsuhiko, Osaka 563 (JP); TANAKA, Shoji, Hyogo 659 (JP)
(74) Representative: Stoner, Gerard Patrick
(86) International application number: JP9000042
(87) International publication number: WO9008190

(56) References cited:
- EP-A- 0 249 412
- WO-A-86/02099
- Chemical Abstracts, volume 101, no. 5, 30 July 1984, (Columbus, Ohio, US), Bradbury, Alan F et al : "Amide forming enzymes in thyroid and pituitary ", see page 218, abstract 35032t, & Pept.Struct.Funct.Proc.Am.Pept.Symp.8th 1983, (), 249-252
- Chemical Abstracts, volume 105, no. 25, 22 December 1986, (Columbus, Ohio, US), Sakata, Junichiro et al : "Tissue distribution and characterization of peptide C-terminal x-amidating activity in rat ", see page 501, abstract 223299s, & Biochem.Biophys.Res.Commun. 1986, 140 (1), 230- 236
- Chemical Abstracts, volume 105, no. 21, 24 November 1986, (Columbus, Ohio, US), Mizuno, Kensaku et al : "Peptide C-terminal x-amidating enzyme purified to homogeneity from Xenopus laevis skin. ", see page 303, abstract 186374q, &Biochem.Biophys.Res.Commun. 1986, 137( 3), 984- 991
- Chemical Abstracts, volume 103, no. 19, 11 November 1985, (Columbus, Ohio, US), Wand, Gary S et al : "Characterization of a peptide alpha-amidation activity in human plasma and tissues ", see page 466, abstract 157942b, & Metab.Clin.Exp.1985, 34(11), 1044-1052
- Chemical Abstracts, volume 109, no. 9, 29 August 1988, (Columbus, Ohio, US), Ohsuye, Kazuhiroet al : "Cloning of cDNA encoding a new peptide C-terminal x-amidating enzyme having a putative membrane-spanning domain fron Xenopus laevis skin", s. p. 175, abstract 67901g, & Biochem.Biophys.Res.Commun. 1988, 150(3), 1275-1281
- Chemical Abstracts, volume 109, no. 15, 10 October 1988, (Columbus, Ohio, US),Mizuno, Kensaku et al : "Peptide C-terminal x-amidated enzyme. Purification and application to the synthesis of x-amidated peptides ", see page 289, abstract 124771r, & Pept.Chem. 1987, (), 427- 430
- Chemical Abstracts, volume 108, no. 15, 11 April 1988, (Columbus, Ohio, US), Mizuno, Kensaku et al : "Cloning and sequence of cDNA encoding a peptide C-terminal x-amidating enzyme from Xenopus laevis ", see page 165, abstract 125517d, & Biochem.Biophys.Res.Commun. 1987, 148( 2), 546- 552
- Chemical Abstracts, volume 103, no. 21, 25 November 1985, (Columbus, Ohio, US), Wand, Gary S et al : "Characterization of peptide alpha-amidation activity in human cerebrospinal fluid and central nervous system tissue ", see page 467, abstract 176005q, & Neuroendocrinology 1985, 41( 6), 482- 489

## Description

### BACKGROUND OF THE INVENTION

### 1. Field of the Invention

The present invention relates to recombinant C-terminal α-amidating enzymes of human thyroid origin and precursors thereof, DNAs coding for these polypeptides, plasmids containing the DNA, host cells transformed with the plasmid, and a process for the production of the enzyme using the transformed cells.

### 2. Description of the Related Art

It is generally known that, in eukaryotic cells, after translation from a messenger RNA (mRNA) some kinds of peptides or proteins are modified by an intracelular enzyme to mature to a natural-type peptide or protein (post-translational modification). But since prokaryotic hosts such as E. coli, which are widely used to produce peptides or proteins of eukaryote origin, cannot carry out a post-translational modification of an expressed peptide or protein, it is sometimes difficult to directly produce a eukaryotic peptide or protein by a recombinant DNA technique using prokaryotic host cells.

One post-translational modification characteristic of eukaryotic cells of peptides or proteins is a modification reaction wherein an α-position of a carboxy terminal (C-terminal) of a peptide or protein is amidated, i.e., -COOH is converted to -CONH₂ , and it is known that many physiologically active peptides or proteins have been subjected to such modification, and that, in some cases the above-mentioned modification of the peptide or protein is essential to the physiological activity thereof. For example, a conversion of the proline amide residue at the C-terminal of natural-type human calcitonin to proline residue decreases the physiological activity thereof to 1/1,600 of the original activity.

Because of the importance of clarifying the mechanism of α-amide formation in tissues, and the promising usefulness of the enzyme for the production of C-terminal α-amidated peptides using, for example, recombinant DNA techniques, many attempts have been made to clarify a detailed mechanism of the biosynthesis of C-terminal α-amidated peptides characteristic to eukaryotic cells. In this clarification first a structure of a precursor of an amidated peptide is clarified from a cDNA analysis of the amidated peptide, and as a result, a general biosynthesis mechanism of such amidated peptides is understood to be that in which mRNA is translated to a precursor of an amidated peptide, which is then amidated at the α-position of the C-terminal thereof by a C-terminal α-amidating enzyme. Note, in the above-mentioned reaction, the precursor of the C-terminal α-amidated peptide as a substrate for a C-terminal α-amidating enzyme is a peptide or protein represented by a general formula R-X-Gly, wherein R represents an amino acid sequence of the N-terminal side of the peptide or protein, X represents an amino acid residue which is to be α-amidated at the C-terminal thereof, and Gly represents a glycine residue.

On the other hand, in porcine pituitary, Bradburg, A.F. et al., Nature 298, 686-688, 1982, first characterized the α-amidating activity converting a synthetic substrate D-Tyr-Val-Gly to D-Tyr-Val-NH₂ , and demonstrated that the C-terminal glycine in the substrate serves as a nitrogen-donor for α-amidation. Also, Eipper et al., Proc. Natl. Acad. Sci. US, 80, 5144-5148, 1983, reported that the α-amidating enzyme derived from the pituitary gland of rat requires copper cation and ascorbate, in addition to molecular oxygen, for its activity. Husain, I. et al., FEBS Lett., 152 227-281, 1983; and Kizer, J. S. et al., Proc. Natl. Acad. Sci. US, 81, 3228-3232, 1984, also reported a C-terminal α-amidating enzyme, but did not report a purified enzyme. Recently, Murthy A.S.N. et al., J. Biol. Chem., 261, 1815-1822, 1986, partially purified a C-terminal α-amidating enzyme from the pituitary gland of cattle, and showed that several types of enzymes having different molecular weights and electric charges are present.

Recently, Mizuno et al. succeeded in isolating a C-terminal α-amidating enzyme in a homogeneous and pure form from a skin of Xenopus laevis (see Mizuno, K. et al., Biochem. Biophys. Res. Commun. 137, 984-991, 1988, and EP 0249412 A3 corresponding to Japanese Patent Application No. 61-131089) and further succeeded in determining an entire primary amino acid sequence of the C-terminal α-amidating enzyme of a skin of Xenopus laevis origin by obtaining and sequencing cDNA; see Mizuno, K. et al., Biochem. Biophys. Res. Commun. 148, 546-552, 1987.

On the other hand, Eipper, B. et al., Mol. Endo. 1, 777-790, 1987, cloned cDNA of a C-terminal α-amidating enzyme of the bovine pituitary gland, and demonstrated that the enzyme of the bovine pituitary gland is clearly different from the above-mentioned enzyme of Xenopus laevis origin.

The above-mentioned reports demonstrate that there are many C-terminal α-amidating enzymes of eukaryote origin, and this suggests that each enzyme has a particular substrate specificity different from that of the others; namely, a particular C-terminal α-amidating enzyme is present for a particular α-amidated peptide, although this has not been fully demonstrated as yet.

On the other hand, if a C-terminal α-amidating enzyme can be produced in a large amount, it becomes possible to produce α-amidated peptides, which are industrially important, in a large amount. Nevertheless, as described above, if various kinds of C-terminal α-amidating enzymes exist, and each enzyme has a particular substrate specificity and is responsible for a biosynthesis of a specific α-amidated peptide or protein, when producing a desired α-amidated peptide or protein, it is important to choose a most suitable C-terminal α-amidating enzyme.

Accordingly, there is a current desire to isolate novel C-terminal α-amidating enzymes other than those of Eipper et al. and Mizuno et al., to determine their structures, and to clarify their substrate specificity, for their use for the production of particular α-amidated peptides.

### SUMMARY OF THE INVENTION

Accordingly, the present invention provides novel C-terminal α-amidating enzymes and a process for the production thereof, as well as cloned genes and expression vectors useful for the production process.

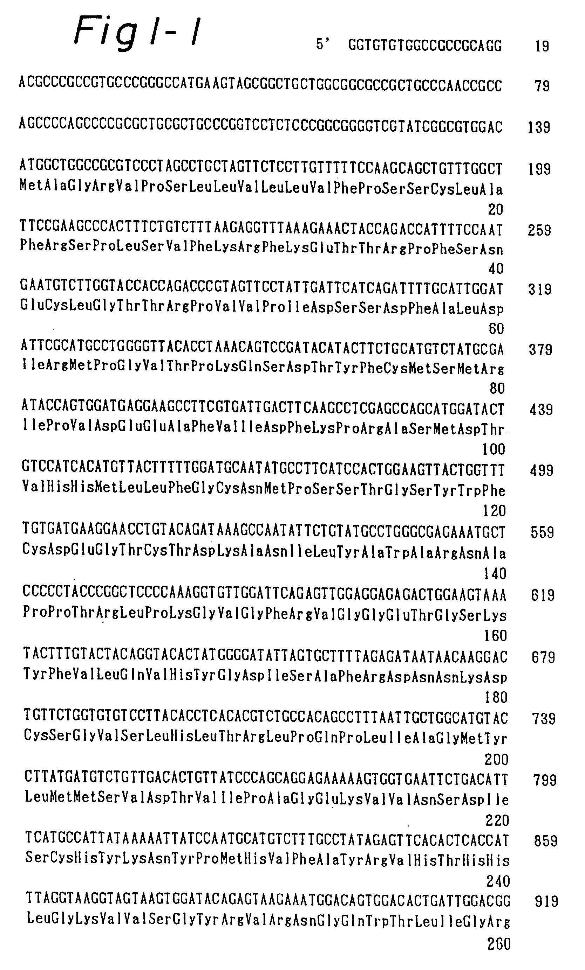

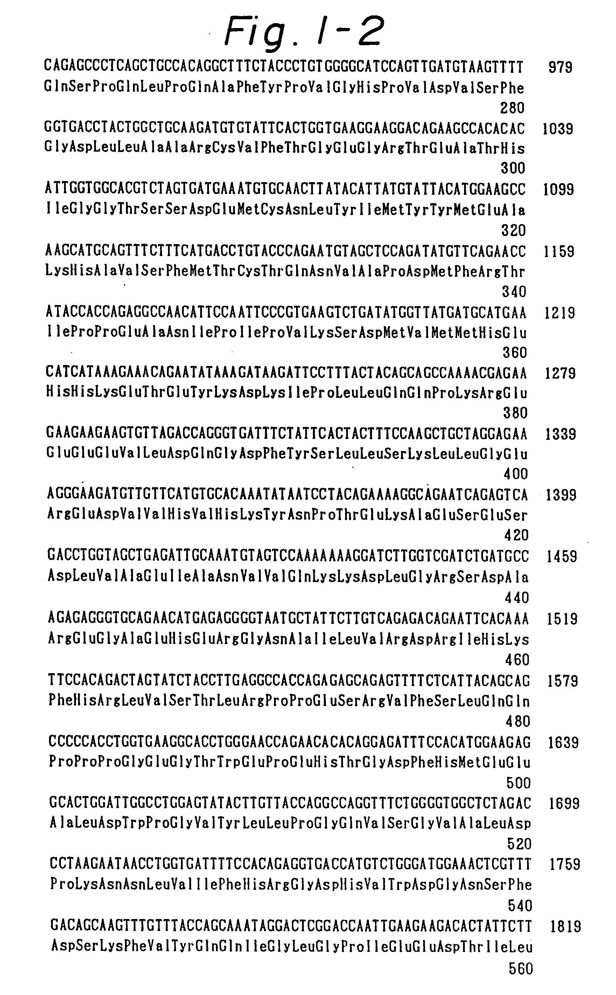

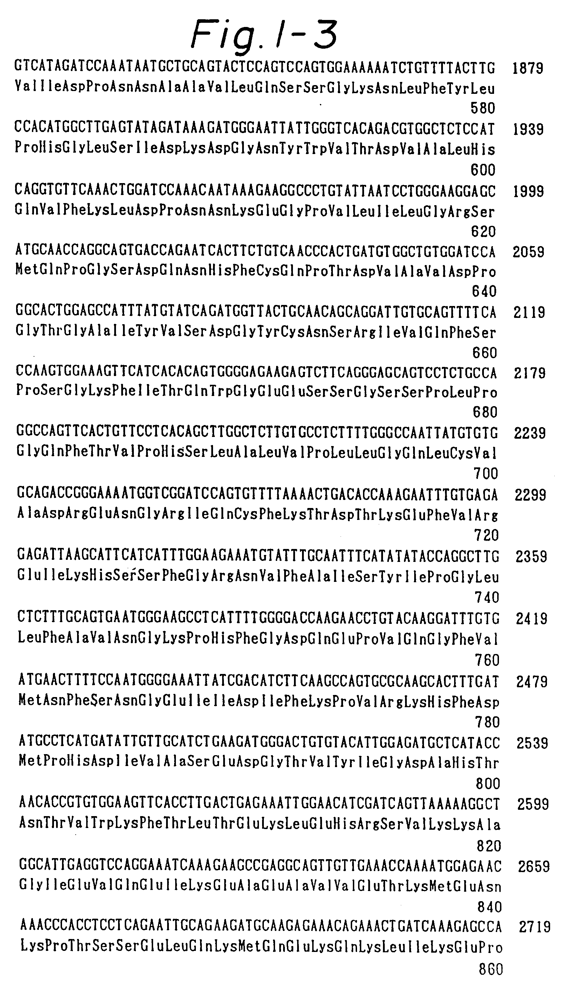

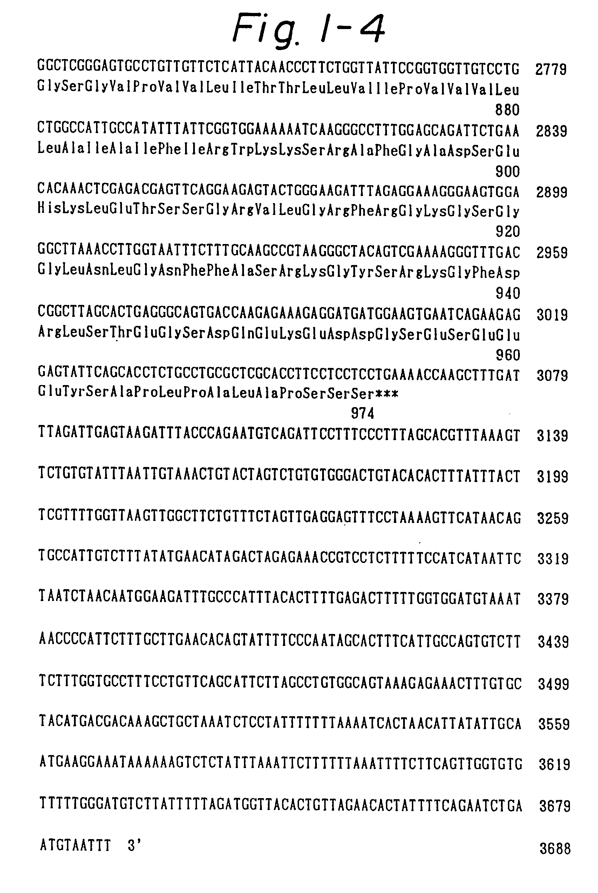

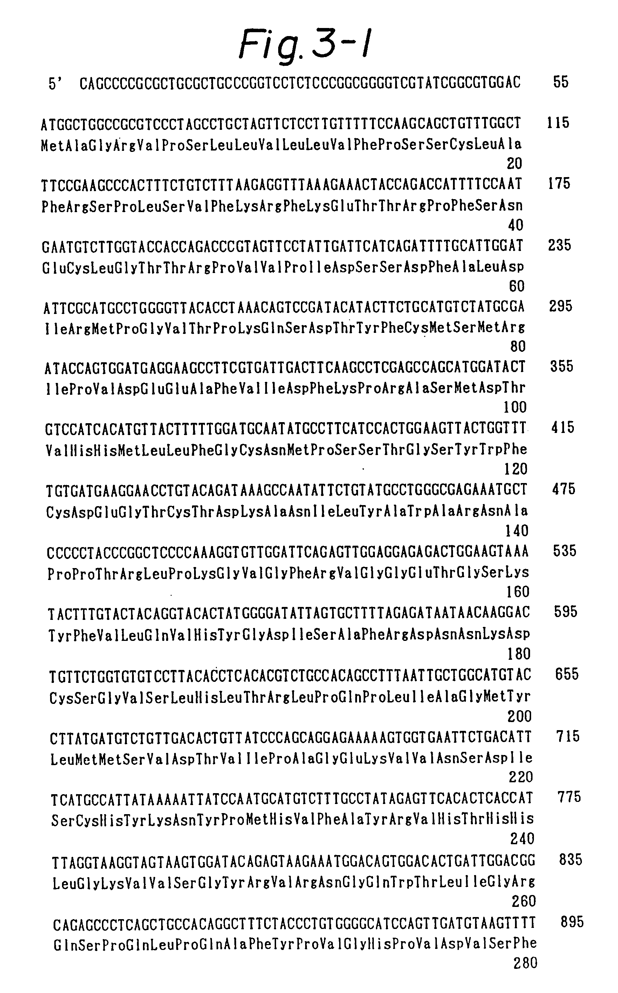

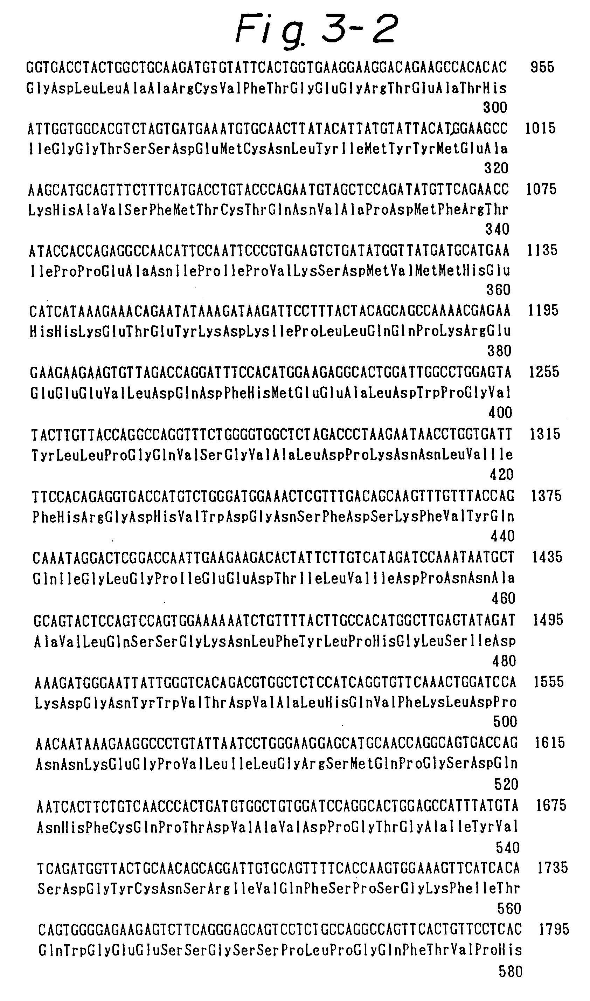

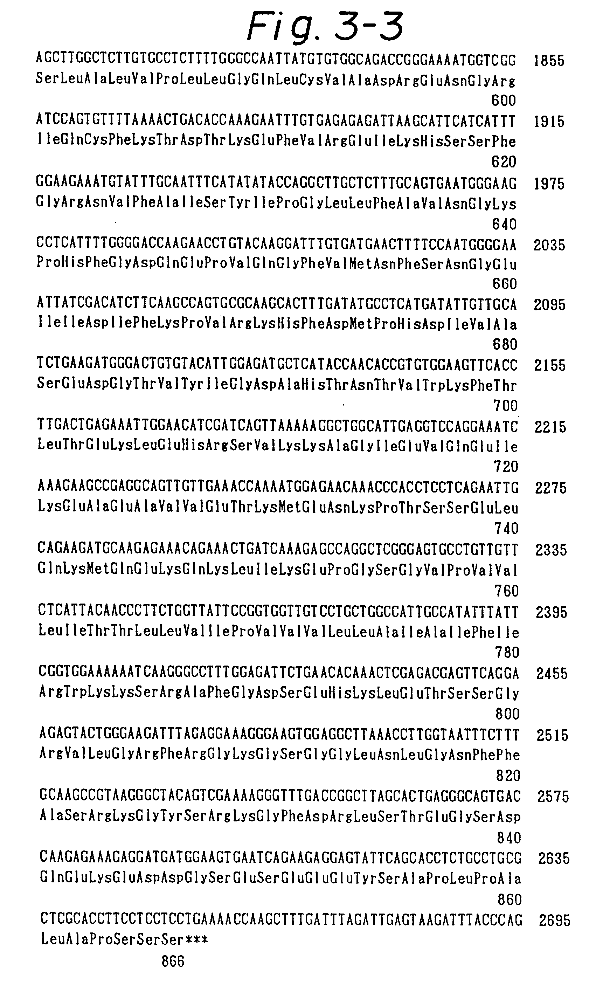

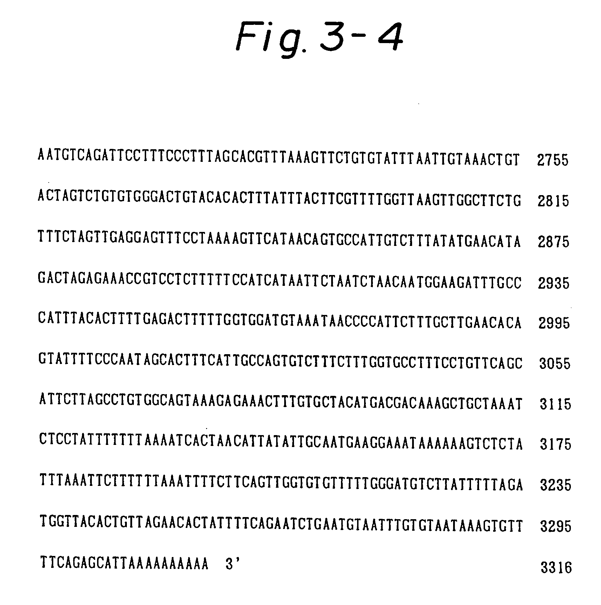

Preferred embodiments of the enzyme and the derivative thereof are, for example,
a C-terminal α-amidating enzyme or biologically active derivative maintaining the enzyme activity thereof comprising an amino acid sequence starting at the 37th Pro and terminating at the 378th Lys in the amino acid sequence shown in Fig. 1; a C-terminal α-amidating enzyme or biologically active derivative maintaining the enzyme activity thereof and having an amino acid sequence starting at an amino acid present between the first Met, inclusive, and the 37th Pro, inclusive, and terminating at an amino acid present between the 378th Lys, inclusive, and the 974th ser, inclusive, in Fig. 1;
a C-terminal α-amidating enzyme or biologically active derivative maintaining the enzyme activity thereof and having an amino acid sequence starting at an amino acid present between the first Met, inclusive, and the 37th Pro, inclusive, and terminating at an amino acid present between the 378th Lys, inclusive, and the 974th Ser, inclusive in Fig. 1, and having an additional C-terminal amino acid or amino acid sequence consisting of up to ten amino acids;
a C-terminal α-amidating enzyme or biologically active derivative maintaining the enzyme activity thereof and having an amino acid sequence starting at an amino acid present between the first Met, inclusive, and the 37th Pro, inclusive, and terminating at an amino acid present between the 378th Lys, inclusive, and the 866th Ser, inclusive, in Fig. 3; and
a C-terminal α-amidating enzyme or biologically active derivative maintaining the enzyme activity thereof and having an amino acid sequence starting at an amino acid present between the first Met, inclusive, and the 37th Pro, inclusive, and terminating at an amino acid present between the 378th Lys, inclusive, and the 866th Ser, inclusive, in Fig. 3, and having an additional C-terminal amino acid or amino acid sequence consisting of up to ten amino acids.

The present invention also provides a process for the production of the above-mentioned C-terminal α-amidating enzyme or biologically active derivatives thereof, comprising the steps of:
(1) Culturing prokaryotic cells or eukaryotic cells transformed with an expression vector containing a gene coding for an enzyme or derivative thereof to produce the enzyme or derivative thereof; and
(2) Recovering the enzyme or derivative thereof from the culture.

The present invention also provides genes coding for the above-mentioned C-terminal α-amidating enzyme or biologically active derivative thereof.

The present invention further provides plasmids and phage vectors containing the above-mentioned gene.

Moreover, the present invention provides prokaryotic cells transfected with the plasmid or eukaryotic cells transfected with the plasmid.

### BRIEF DESCRIPTION OF THE DRAWINGS

Figure. 1-1 to 1-4 represent a nucleotide sequence of a cDNA region in a phage clone λgt10ch201, and a corresponding amino acid sequence;
Figs 2-1 to 2-3 represent a nucleotide sequence of a cDNA region in a phage clone λgt10chT2, and a corresponding amino acid sequence;
Figs 3-1 to 3-4 represent a nucleotide sequence of a cDNA region in a phage clone λgt10ch101, and a corresponding amino acid sequence;
Fig. 4 represents a restriction enzyme cleavage map of the cDNA region in the λgt10ch201 (a), and a strategy for the determination of a nucleotide sequence of the cDNA region (b);
Fig. 5 represents a restriction enzyme cleavage map of the cDNA region in the λgt10chT2(a), and a strategy for the determination of a nucleotide sequence of the cDNA region(b);
Fig. 6 represents a restriction enzyme cleavage map of the cDNA region in the λgt10chT101 (a), and a strategy for the determination of a nucleotide sequence of the cDNA region(b);
Fig. 7 represents the relationship between cDNA regions in the λgt10chT201, λgt10chT2 and λgt10chT101 as well as λgt11chT40 and λgt11chT28 compared with cDNA in a plasmid pXA799 derived from the Xenopus laevis cDNA library;
Fig. 8 represents a process for the construction of a plasmid pUC18chT201 HindIII-BglII;
Fig. 9 represents a process for the construction of a plasmid pUC19chT201 MPF;
Fig. 10 represents a process for the construction of a plasmid P_{L}chT201 MPF-Sph;
Fig. 11 represents a process for the construction of a plasmid P_{L}chT201 MPF-S/B;
Fig. 12 represents a process for the construction of a plasmid pUC19chT201-EcoRI 0.7;
Fig. 13 represents a process for the construction of a plasmid pUC18-Sal;
Fig. 14 represents a process for the construction of a plasmid P_{L}chT201 MPF-Sal;
Fig. 15 represents a process for the construction of a plasmid P_{L}chT201 MPF-Bcl;
Fig. 16 represents a process for the construction of a plasmid pUC19chT201 MFK;
Fig. 17 represents a process for the construction of a plasmid P_{L}chT201 MFK-Sph;
Fig. 18 represents a process for the construction of a plasmid P_{L}chT201 MFK-S/B;
Fig. 19 represents a process for the construction of a plasmid P_{L}chT201 MFK-Sal;
Fig. 20 represents a process for the construction of a plasmid P_{L}chT201 MFK-Bcl;
Fig. 21 represents a process for the construction of plasmids P_{L}chT2 MPF-Pst and P_{L}chT2 MFK-Pst;
Fig. 22 represents a process for the construction of plasmids P_{L}chT2 MPF-Bcl and P_{L}chT2 MFK-Bcl;
Fig. 23 represents the productivity, in terms of substrate amidation ratio (%), of a C-terminal α-amidating enzyme by Escherichia coli W3100 transformed with different plasmid described above;
Fig. 24 represents a process for the construction of a plasmid pUC18-HpaII;
Fig. 25 represents a process for the construction of plasmids pKDch201 MPF-Sph, -Sal, -S/B and -Bcl;
Fig. 26 represents a process for the construction of a plasmid pKDchT201-PstI;
Fig. 27 represents a process for the construction of a plasmid pUC18chT2 HindIII-BglII;
Fig. 28 represents a process for the construction of a plasmid pUC18chT201-chT2;
Fig. 29 represents a process for the construction of a plasmid P_{L}chT2 KpnI-PstI;
Fig. 30 represents a process for the construction of a plasmid pKDchT2 PstI;
Fig. 31 represents a process for the construction of a plasmid pKDchT2-Bcl;
Figs. 32(a) and 32(b) represent graphs showing the productivity, in terms of substrate amidation ratio (%), of a C-terminal α-amidating enzyme by COS-1 cells transfected with different plasmids described above;
Figs. 33(a), 33(b) and 34 schematically shown amino acid sequences of C-terminal α-amidating enzymes derived from various plasmids.

### DESCRIPTION OF THE PREFERRED EMBODIMENTS

The present inventors have already succeeded in isolating two kinds of cDNAs coding for C-terminal α-amidating enzymes, from the skin of Xenopus laevis, which were cloned in plasmids pXA 457 and pXA 799. E. coli DH1/pXA799 containing the plasmid pXA799 was deposited with the Fermentation Research Institute, Agency of Industrial Science and Technology (FRI), 1-3, Tsukuba-shi 1-chome, Ibaraki-ken, 301 Japan, as FERM BP-1586, on December 3, 1987; (Japanese Patent Publication No. 1-104168; EP 0299790 A2). Moreover, by comparing a primary amino acid sequence encoded by cDNA for a C-terminal α-amidating enzyme of the bovine pituitary gland cloned by Eipper et al., supra, with primary amino acid sequences encoded by the above-mentioned cDNAs for Xenopus laevis C-terminal α-amidating enzymes cloned by the present inventors, it is found that the amino acid sequences of an N-terminal region, which was proven to be essential for the enzyme activity, are similar in all the C-terminal α-amidating enzymes of different origins. This suggests that, even though there are various kinds of C-terminal α-amidating enzymes in eukaryotic cells, a primary amino acid sequence of a domain essential for the enzyme activity is conserved between the species and tissues. On the basis of this concept, the present inventors attempted to clone cDNAs coding for novel C-terminal α-amidating enzymes by screening cDNA banks derived from RNAs of different origins, using as a probe the cDNA coding for a C-terminal α-amidating enzyme of Xenopus laevis origin.

In this case, considering that human calcitonin, a well known C-terminal amidated peptide, is biosynthesized in the thyroid gland, the present inventors extracted mRNA from the human thyroid gland, prepared cDNA library, and screened the library using the above-mentioned cDNA derived from Xenopus laevis as a probe. As a result, two kinds of cDNAs coding for novel C-terminal α-amidating enzymes having primary amino acid sequences clearly different from those of the above-mentioned known C-terminal α-amidating enzymes, were obtained. Moreover, the cDNAs thus cloned were used to transform E. coli or transfect animal cells, COSl (ATCC CRL 1680; Cell, 23, 175-182, 1981) to express the cloned genes, and it was confirmed that the expression products exhibit the activity of a C-terminal α-amidating enzyme as determined using a synthetic substrate [¹²⁵I] Ac-Tyr-Phe-Gly.

According to the present invention, the C-terminal α-amidating enzyme catalyzes a conversion of a peptide or protein represented by the formula: R-X-Gly wherein X is an amino acid to be α-terminal α-amidated, Gly represents glycine and R represents the remaining part of the peptide or protein, to a peptide or protein represented by the formula: R-X-NH₂. The present C-terminal α-amidating enzyme includes, in addition to native enzymes of human thyroid gland origin, modified enzymes wherein one or more than one amino acid on an amino acid sequence of the native enzyme is replaced by one or more than one different amino acid, wherein one or more than one amino acid is added to the amino acid sequence of the native enzyme, or wherein one or more than one amino acid is deleted from the amino acid sequence of the native enzyme, white maintaining the above-mentioned enzyme activity.

Next, the present invention is explained in more detail.

### (1) Isolation of cDNA coding for C-terminal α-amidating enzyme

Total RNA was prepared from human thyroid gland, and from the total RNA, poly(A) RNA was prepared using an oligo(dT) cellulose column. Next, the poly (A) RNA was used to prepared a cDNA according to a procedure of Gubler et al. (Gubler; V. and Hoffman. B. J., Gene 25, 263, 1983), and the cDNA thus obtained was used to prepare a λgtII phage cDNA library. A plasmid pXA799 containing cDNA coding for a C-terminal α-amidating enzyme of Xenopus laevis was digested with ApaI and DraI, and the resulting ApaI-DraI DNA fragment of about 2.8 kb was labeled with (α-³²P]dCTP by nick translation to prepare a DNA probe. Next, the above-mentioned λgtII cDNA library was screened using the DNA probe, to obtain phage clones λgt11chT28 and λgt11ch40. As a result of analysis of these two clones, it was found that these clones do not contain a cDNA coding for the entire C-terminal α-amidating enzyme.

Accordingly, to obtain a cDNA coding for the entire C-terminal α-amidating enzyme, a phage λgt10 cDNA library was prepared from the above-mentioned poly (A) RNA, by the same procedure as described above. The λgt10 cDNA library was screened using the above-mentioned ApaI-PraI DNA probe derived from pXA799, to obtain a phage clone λgt10ch T2. Moreover, the cDNA library was again screened using a DNA probe derived from a cDNA region in the above-mentioned λgt11chT28 clone, to obtain a phage clone λgt10chT201. In addition, the cDNA library was screened using a DNA probe which was a labeled SmaI-XhoI DNA fragment corresponding to a region from the 35th G to the 425th A in the λgt10ch201, to obtain a phage clone λgt10ch101.

### (2) Analysis of cDNAs coding for C-terminal α-amidating enzyme

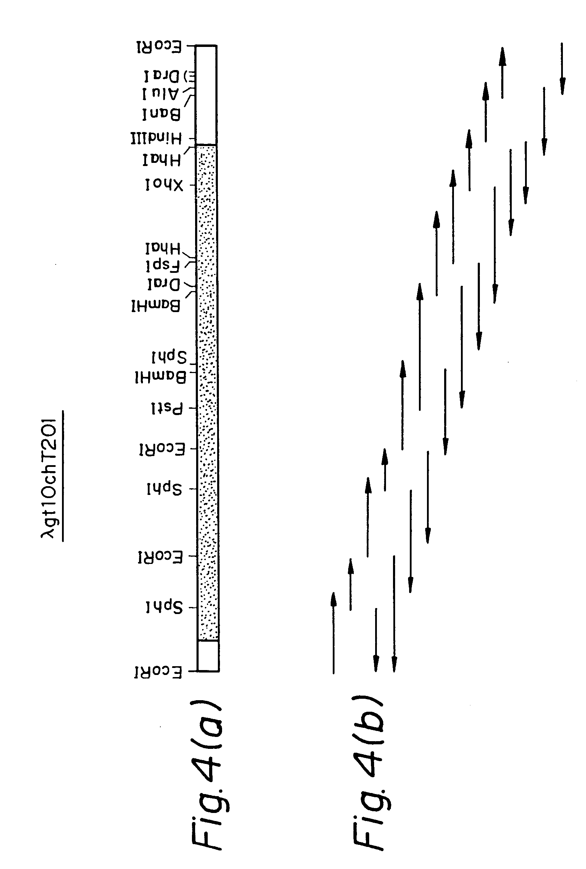

The phage DNAs obtained as described above, i.e., λgt11chT28, λgt11chT40, λgt10chT201, λgt10chT2, and λgt10chT101, were cleaved with various restriction enzymes to prepare their restriction enzyme cleavage maps. The maps for cDNA regions in the phages λgt10chT201, λgt10chT2, and λgt10chT101 are shown in Figs. 4(a), 5(a), and 6(a), respectively. Next, each DNA fragment prepared by cleaving the λgt10chT201, λgt10chT2, and λgt10chT101 with various restriction enzymes was subcloned in M13 phage to determine the nucleotide sequence, according to a procedure of Sanger et al. (Sanger, F. et al., Proc. Natl. Acad. Sci. U.S.A. 34, 5463-5467, 1977), and an entire nucleotide sequence of the cDNA region in the phage clone was assembled according to a strategy shown in Figs. 4(b), 5(b) or 6(b). The nucleotide sequences of the cDNA region in the phage clones λgt10chT201, λgt10chT2, and λgt10chT101 are shown in Figs. 1, 2, and 3, respectively.

### (a) Analysis of chT201 cDNA

The analysis of the cDNA region in the phage clone λgt10chT201 (designated chT201 cDNA) revealed the following:
(1) The chT201 cDNA consists of 3688 base pairs and contains a long reading frame starting with the 140th nucleotide and ending at the 3061th nucleotide (according to the nucleotide number shown in Fig. 1);
(2) An amino acid sequence corresponding to the open reading frame consists of 974 amino acid residues starting with the N-terminal Met-Ala-Gly- and ending with the C-terminal -Ser-Ser-Ser;
(3) In comparison with an amino acid sequence encoded by cDNA in pXA799 derived from Xenopus laevis, the amino acid sequence encoded by the ch201 cDNA contains an additional amino acid sequence consisting of 107 amino acid residues starting with the 388th amino acid and ending at the 494th amino acid (according to the amino acid number shown in Fig. 1); and the homology between both amino acid sequences, excluding the above-mentioned additional amino acid sequence, is about 54% by DNA level, and about 65% by protein level;
(4) The amino acid sequence encoded by the chT201 cDNA contains an amino acid sequence represented by the sequence Asn-X-Ser, wherein X is any amino acid corresponding to the 411 to 413th amino acids, and the amino acid sequence represented by the sequence Asn-X-Thr, wherein X is any amino acid sequence corresponding to the 762-764th amino acids. Since these amino acid sequences are considered to be N-glycosylation sites from analyses of many other glycoprotein, there is a great possibility that the protein encoded by the chT201 cDNA is N-glycosylated;
(5) Since the amino acid sequence encoded by the chT201 cDNA contains a hydrophobic amino acid sequence near the N-terminus thereof and the hydrophobic sequence is considered to be a signal sequence characteristic to a secretary protein, the C-terminal α-amidating enzyme is believed to be expressed as a precursor protein, which is then processed to form a mature protein; and,
(6) The amino acid sequence encoded by the chT201 cDNA contains a region starting from the 864th amino acid and ending at the 911th amino acid, in which hydrophobic amino acids continuously occur, and this region is immediately followed by basic amino acids, i.e., arginine and lysine. Since this type of structure is frequently found in a trancemembrane domain of a membrane protein, there is a great possibility that the present C-terminal α-amidating enzyme is present as a membrane protein.

### (b) Analysis of chT2 cDNA

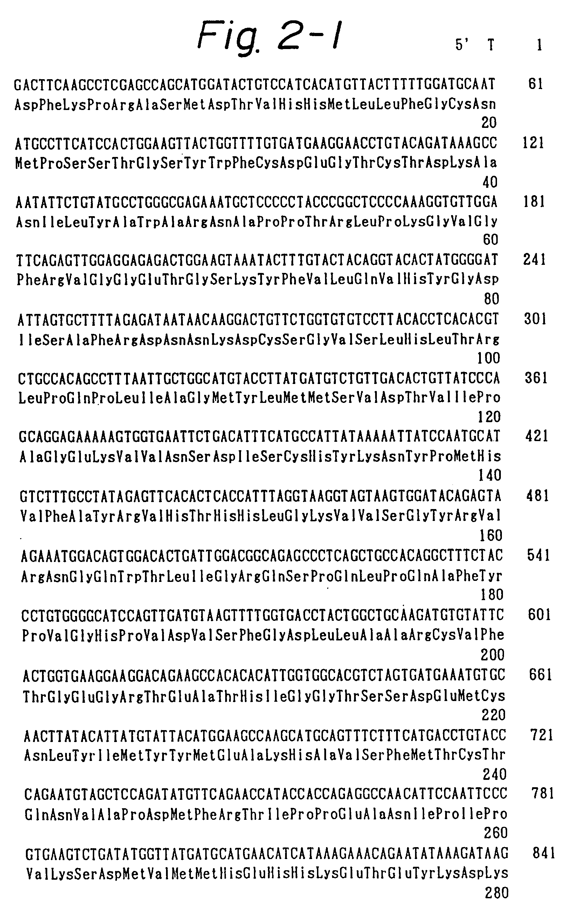

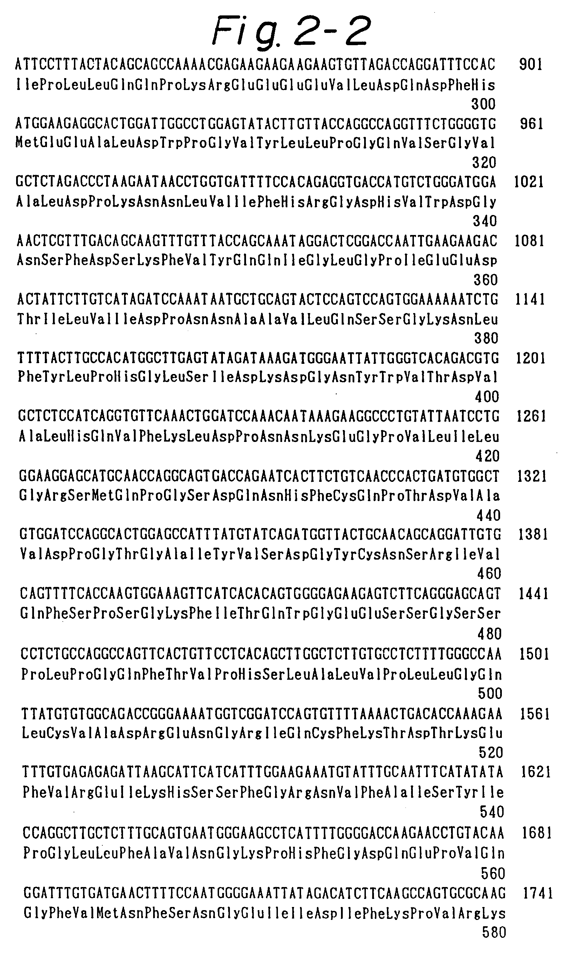

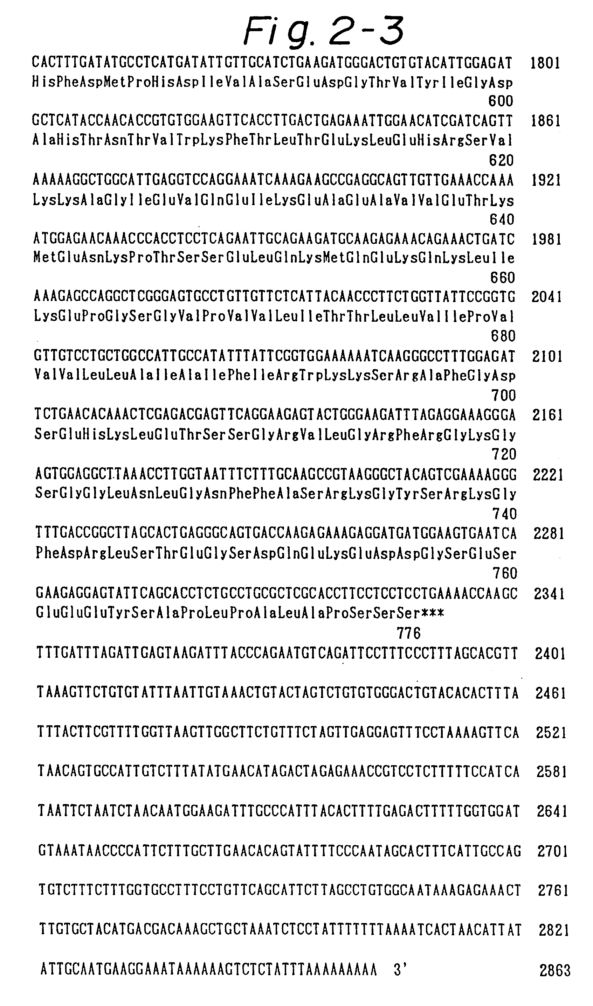

The analysis of the cDNA region in a phage clone λgt10chT2 (designated chT2 cDNA) revealed the following:
(1) The chT2 cDNA consists of 2863 base pairs wherein the first nucleotide T corresponds to the 409th nucleotide in the chT201 cDNA, and contains a long reading frame starting at the second nucleotide and ending at the 2329th nucleotide (according to the nucleotide number shown in Fig. 2;
(2) An amino acid sequence corresponding to the open reading frame consists of 776 amino acid residues starting at the N-terminal Asp-Phe-Lys- and ending at the C-terminal -Ser-Ser-Ser, wherein the N-terminal Asp-Phe-Lys- corresponds to the -Asp-Phe-Lys- sequence starting at the 91th amino acid in the amino acid sequence encoded by the chT201 cDNA;
(3) In comparison with the amino acid sequence encoded by the chT201, the amino acid sequence encoded by the chT2 lacks an amino acid sequence corresponding to an amino acid sequence portion consisting of 107 amino acid residues starting at the 388th amino acid and ending at the 494th amino acid (according to the amino acid number shown in Fig. 1) in the amino acid sequence encoded by the chT201 cDNA. Note, the amino acid sequence consisting of the 107 amino acid residues is also absent from the amino acid sequence encoded by the cDNA in the pXA799. Moreover, the amino acid sequence encoded by the chT2 cDNA lacks an amino acid corresponding to the 897th amino acid Ala (according to the amino acid number shown in Fig. 1) in the amino acid sequence encoded by the chT201 cDNA. Namely, the amino acid sequence encoded by the chT2 cDNA is the same as the amino acid sequence encoded by the chT201 cDNA, except that the former lacks an amino acid sequence and an amino acid corresponding to the amino acid sequence portion consisting of the above-mentioned 107 amino acid residues and the 897th amino acid residue Ala of the latter.

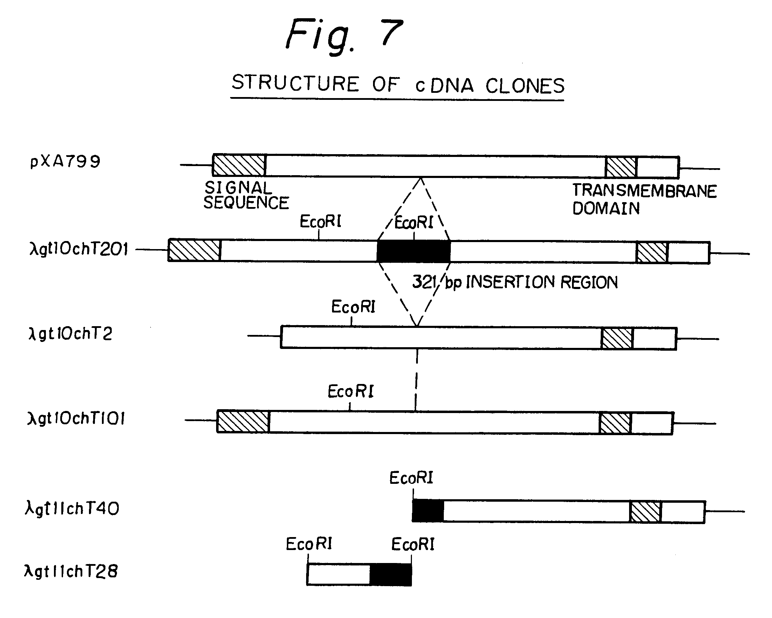

Therefore, the chT2 cDNA was an incomplete cDNA in that the chT2 cDNA lacks a 5'-terminal region corresponding to the 5'-terminal region up to the codon for the 90th Ile (see Figs. 2 and 7).

### (c) Analysis of chT101 cDNA

The analysis of the cDNA region in the phage clone λg10chT101 (designated chT101 cDNA) revealed the following:
(1) The chT101 cDNA consists of 3316 base pairs and contains a long reading frame starting at the 56th nucleotide and ending at the 2653th nucleotide;
(2) An amino acid sequence corresponding to the open reading frame consists of 866 amino acid residues starting at the N-terminal Met-Ala-Gly- and ending at the C-terminal -Ser-Ser-Ser;
(3) In comparison with the amino acid sequence encoded by the chT201 cDNA, the amino acid sequence encoded by the chT101 cDNA is the same as that encoded by the chT201, except that the former lacks an amino acid sequence corresponding to an amino acid sequence portion consisting of 107 amino acid residues starting at the 388th amino acid and ending at the 494th amino acid, and an amino acid corresponding to the 897th amino acid residue Ala of the latter. On the other hand, in comparison with the amino acid sequence encoded by the chT2 cDNA, the amino acid sequence encoded by the chT2 cDNA corresponds to an amino acid sequence starting at the 91th Asp and ending at the last amino acid Ser of the amino acid sequence encoded by the chT101 cDNA. Therefore, the chT101 cDNA completely supplements a 5'-region lacking in the chT2 cDNA.

From the above-mentioned analysis of the chT201 cDNA, chT2 cDNA, and chT101 cDNA, it was assumed that the C-terminal α-amidating enzymes of human thyroid origin can be classified into two types; one represented by an enzyme encoded by the chT2 cDNA or chT101 cDNA, and another represented by an enzyme encoded by the chT201 cDNA. It is assumed that the chT201-type enzyme contains two N-glycosylation sites and that the chT201-type enzyme contains one N-glycosylation site, because one of the two possible N-glycosylation sites in the ch201-type enzyme is present in the additional region consisting of 107 amino acids not present in the chT2-type enzyme.

Nevertheless, so far enzyme proteins predicted from the chT201 cDNA and chT101 cDNA have not been isolated from the human thyroid, and therefore, the biosynthetic mechanism, including the processing of the N- and/or C-terminal portion and glycosylation, of protein corresponding to the chT201 cDNA and chT101 cDNA is not clear. Accordingly, to resolve there questions, the chT201 cDNA and chT101 cDNA were shortened to obtain various cDNA derivatives, which were then introduced into prokaryotic cells and eukaryotic cells to produce various modified proteins, and the modified proteins were assayed to determine their C-terminal α-amidating activity, as described hereinafter.

### (3) Expression of C-terminal α-amidating enzyme derivatives in prokaryotic cells

Where C-terminal α-amidating enzyme derivatives are expressed in prokaryotic cells such as E. coli cells, the position of the N-terminus in the chT201 cDNA, chT2 cDNA or chT101 cDNA should be chosen. Recently, Eipper et al. (Eipper, B. et al., Mol. Endo. 1, 777-790, 1987) cloned cDNA coding for a C-terminal α-amidating enzyme of bovine origin, and determined a primary amino acid sequence of the enzyme. There is an approximately 92% homology between an amino acid sequence encoded by the chT201 cDNA and that encoded by the cDNA of bovine origin at the amino acid sequence level. Considering the analysis of the cDNA of bovine origin by Eipper et al., and the analysis of the pXA799 and pXA457 of Xenopus laevis by the present inventors, it is assumed that a processing site of a protein encoded by the chT201 cDNA or chT101 cDNA is between the 30th Arg and the 31th Phe, or between the 36th Arg and the 37th Pro. Accordingly, the enzyme derivatives were designed such that the N-terminus thereof was at the 31th Phe or the 37th Pro in the amino acid sequence shown in Figs. 1 or 3. Moreover, the enzyme derivatives were designed such that the C-termini thereof were at different sites in the amino acid sequence shown in Figs. 1 or 3, although in some case the derivative has at its C-terminus an additional amino acid sequence derived from a multiple cloning site in a plasmid used to construct expression plasmid. DNAs coding for chT201-type enzyme derivatives were constructed from the chT201 cDNA; and DNAs coding for chT101-type enzyme derivatives were constructed by joining a 5'-terminal side of the chT201 cDNA and a 3'-terminal side of the chT2 cDNA rather than from the chT101 cDNA, from the viewpoint of convenience of the construction.

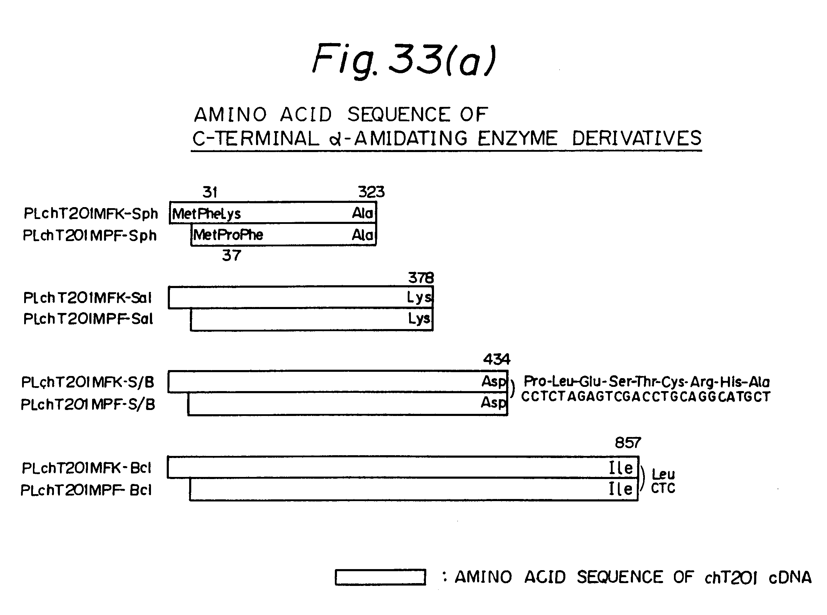

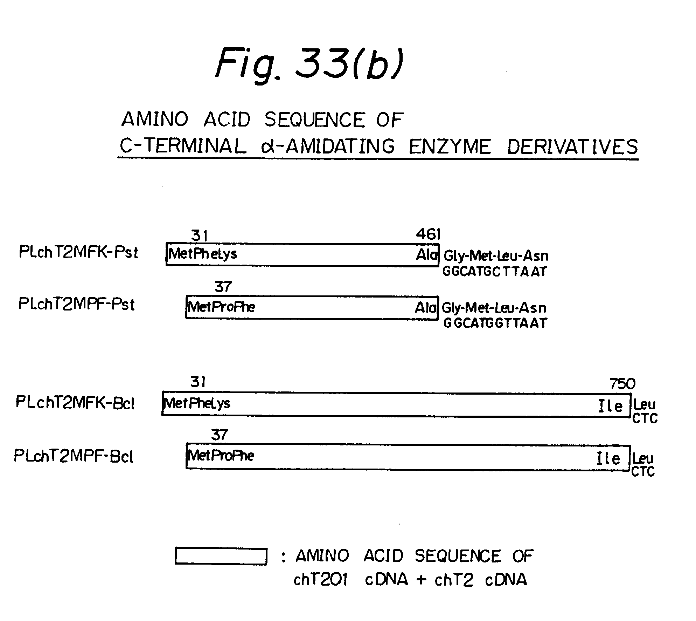

Figures 33(a) and 33(b) schematically represent enzyme derivatives designed for the expression in E. coli. The enzyme derivatives (chT201-type derivatives) shown in Fig. 33(a) start at the 31th Phe or 37th Pro and end at the 323th Ala, the 378th Lys, the 434th Asp or 857th Ile in the amino acid sequence shown in Fig. 1; although all derivatives have at their N-terminus an additional Met corresponding to a translation start codon, and at their C-terminus, some derivatives have an additional amino acid or amino acid sequence derived from a multicloning site present in a plasmid used for the construction of corresponding expression vectors. The C-terminal additional amino acid and amino acid sequence are shown outside the boxes in Fig. 33(a). The enzyme derivatives (chT101-type derivatives) shown in Fig. 33(b) start at the 31th Phe or the 37th Pro and end at the 461th Ala or the 750th Ile in the amino acid sequence shown in Fig. 3; although all derivatives have an additional amino acid Met corresponding to a translation start codon, at their N-terminus, and at their C-terminus, some derivatives have an additional amino acid or amino acid sequence derived from a multicloning site present in a plasmid used to construct corresponding expression vectors. The C-terminal additional amino acid and amino acid sequence are shown outside the boxes in Fig. 33(b).

In the expression of the enzyme derivatives designed as above, expression vectors P_{L}chT201 MPF-Sph, P_{L}chT201 MPF-Sal, P_{L}chT201 MPF-S/B, P_{L}chT201 MPF-Bcl, P_{L}chT201 MFK-Sph, P_{L}chT210 MFK-Sal, P_{L}chT201 MFK-S/B, and P_{L}chT201 MFK-Bcl were constructed for the expression of the chT201-type derivatives, and P_{L}chT2 MPF-Pst, P_{L}chT2 MPF-Bcl, P_{L}chT2 MFK-Pst, and P_{L}ch MFK-Bcl were constructed for the expression of the chT101 type derivatives. Note, in Figs. 33(a) and 33(b), the enzyme derivatives are identified by the names of the expression vectors. These expression vectors contain a cDNA coding for the chT201-type protein derived from chT201 cDNA, or cDNA coding for the chT101-type protein derived from chT201 cDNA and chT2 cDNA under the control of a P_{L} promoter of λ phage origin. These expression vectors were constructed from the phage clones λgt10chT201 and λgt10chT2, commercially available plasmids pUC18 and pUC19, and a plasmid pUCP_{L} CI. Note, E. coli transformed with the plasmid pUCP_{L} CI was designated as E. coli SBM305, and deposited with the FRI under the Budapest treaty as FERM BP-2240, on January 11, 1989. A construction process for the pUCP_{L} CI is described in Japanese Patent Application No. 62-306867 (EP 0299790 A2).

These expression vectors can be used to transform a host such as E. coli. For example, E. coli W3110 was transformed to obtain the corresponding transformants, W3110/P_{L}chT201 MPF-Sph, W3110/P_{L}chT201 MPF-Sal, W3110/P_{L}chT201 MPF-S/B, W3110/P_{L}chT201 MPF-Bcl, W3110/P_{L}chT201 MFK-Sph, W3110/P_{L}chT201 MFK-Sal, W3110/P_{L}chT201 MFK-S/B, W3110/P_{L}chT201 MFK-Bcl, W3110/P_{L}chT2 MPF-Pst, W3110/P_{L}chT2 MPF-Bcl, W3110/P_{L}chT2 MFK-Pst, and W3110/P_{L}chT2 MFK-Bcl.

Then the transformants were separately cultured in a conventional medium to obtain cultured cells, which were sonicated to recover an expression product in a precipitated fraction.

### (4) Expression of C-terminal α-amidating enzyme derivatives in eukaryotic cells

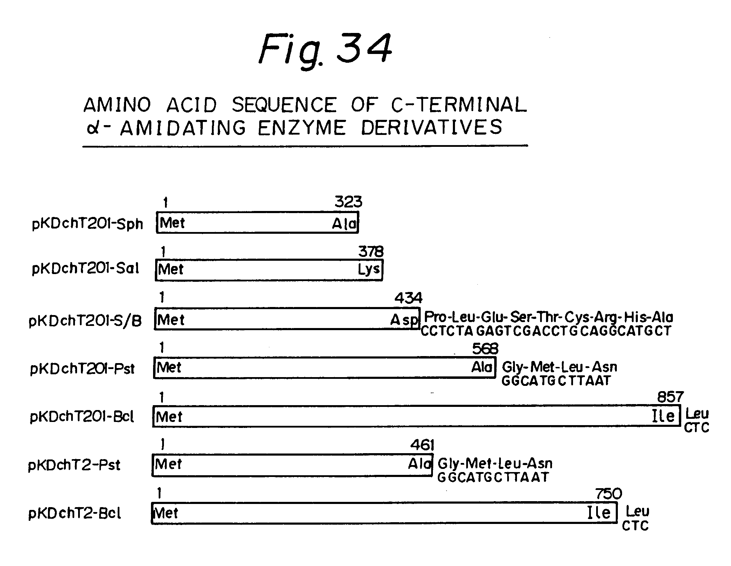

For the expression in eukaryotic cells, chT201-type enzyme derivatives starting at the first amino acid Met and ending at the 323rd Ala, the 378th Lys, the 434th Asp, the 568th Ala or the 857th Ile in the amino acid sequence shown in Fig. 1, and chT101-type enzyme derivatives starting at the first Met and ending at the 461th Ala or the 750th Ile in the amino acid sequence in Fig. 3, (although some derivatives have an additional amino acid or amino acid sequence) were designed. These enzyme derivatives are schematically shown in Fig. 34. In this Figure, the additional amino acid and amino acid sequences derived from multicloning site present in a plasmid used for the construction of expression vectors are shown outside the boxes.

To express the above-designed enzyme derivatives in eukaryotic cells such as COSl cells, expression plasmids pKDchT201-Sph, pKDchT201-Sal, pKDchT201-S/B, pKDchT201-Pst, pKDchT201-Bcl, pKDchT201-Pst, and pKDchT2-Bcl were constructed from the phage clones λgt10chT201 and λgt10chT2, commercially available plasmids pUC18 and pUC19, and a plasmid pKD. Note, E. coli transformed with the plasmid pKD was designated as E. coli SBM303 and deposited with the FRI under the Budapest treaty as FERM BP-2238, on January 11, 1989. Note, in Fig. 34, the enzyme derivatives are identified by the names of the expression vectors. These expression vectors contain a cDNA insert coding for a desired enzyme derivative under the control of the early promoter derived from SV40 virus. Further, these expression vectors were used to transfect animal cells such as COSl cells, and the transfected cells cultured in a conventional medium. The desired enzyme derivative was then recovered from a culture medium.

### (5) Assay of enzyme activity

A C-terminal α-amidating enzyme activity is assayed to confirm that a protein expressed in a transfected host is a C-terminal α-amidating enzyme, and that when a C-terminal α-amidating enzyme acts on the substrate thereof, an amidated peptide or protein is produced. For an assay of an expression of an enzyme in E. coli, E. coli cells are disrupted and the disruptant is centrifuged to obtain a precipitate containing a major portion of the expression product, the precipitate is solubilized with 6 M guanidine hydrochloride, and the solution thus obtained is dialyzed to obtain an assay sample.

Enzyme activity is assayed by using a reaction wherein a substrate generally represented by R-X-Gly is converted to R-X-NH₂ , for example, a reaction wherein a synthetic substrate [¹²⁵I] -Ac-Tyr-Phe-Gly is converted to [¹²⁵I] -Ac-Tyr-Phe-NH₂.

Namely, first a labeled substrate (labeled R-X-Gly) is subjected to a reaction with a test enzyme solution in Tris-HCl buffer, to this reaction mixture are added Tris-HCl buffer and ethyl acetate, and after mixing, the whole is centrifuged to separate an organic phase and an aqueous phase. Since a major portion of the unreacted labeled substrate (labeled R-X-Gly) remains in an aqueous phase, and an amidated labeled products (labeled R-X-NH₂) transfers to an organic phase, the substrate and the product can be easily separated.

In examples of the present invention, a C-terminal α-amidating enzyme of the present invention was assayed using synthetic peptide [¹²⁵I] -Ac-Tyr-Phe-Gly as a substrate according to the following procedure. [¹²⁵I] -Ac-Tyr-Phe-Gly (1 pmole, 70,000-150,000 cpm) was incubated with an enzyme preparation, in a final volume of 250 µl containing 0.2 M Tris-HCl buffer (pH 7.0), 2 µM CuSO₄ , 0.25 mM ascorbic acid, 25 µg catalase (Boehringer), 0.1% Lubrol (PX type, Nakarai Chemicals). The reaction mixture was kept at 37°C for 1 to 4 hours, and then 0.75 ml of 1 M Tris-HCl buffer (pH 7.0) and 2 ml of the organic phase of an ethyl acetate/water mixture was added. The two phases were mixed vigorously in a Vortex mixer, and after centrifugation at 3000 rpm for 3 mins, the organic phase thus separated was transferred to another test tube. The radioactivity in the organic and aqueous layers was measured by a gamma scintillation counter. Under the conditions described above, over 98% of the radioactivity of the authentic [¹²⁵I] -Ac-Tyr-Phe-Gly was retained in an aqueous phase and over 98% of the radioactivity of the authentic [¹²⁵I] -Ac-Tyr-Phe-NH₂ was transferred to an organic phase.

The yield of conversion is calculated from the ratio of the radioactivity in an organic phase such as an ethyl acetate phase to the total radioactivity. In this assay, one unit is defined as the enzyme activity that gives fifty percent conversion of 1 p mole substrate, such as [¹²⁵I] -Ac-Try-Phe-Gly, to [¹²⁵I] -Ac-Tyr-Phe-NH₂ for 1 hour.

### (6) Protein derivatives exhibiting enzyme activity

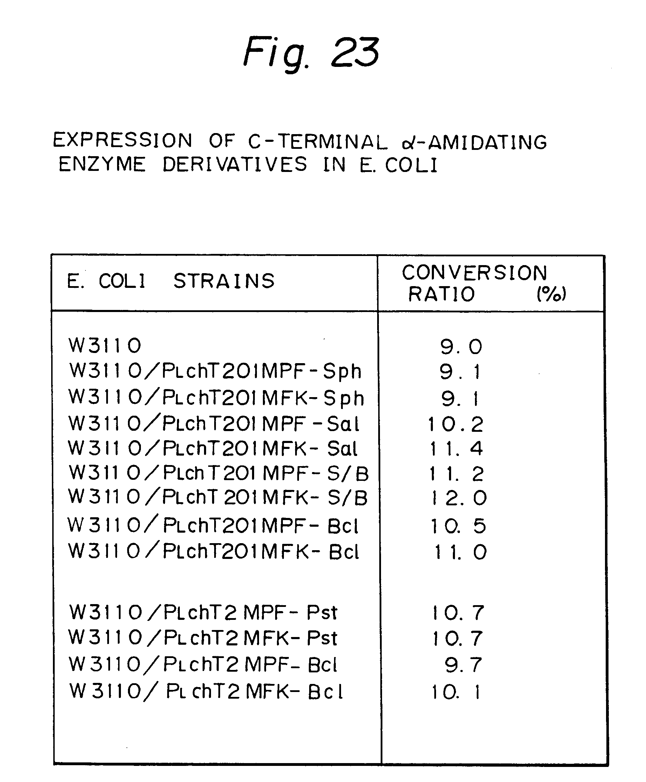

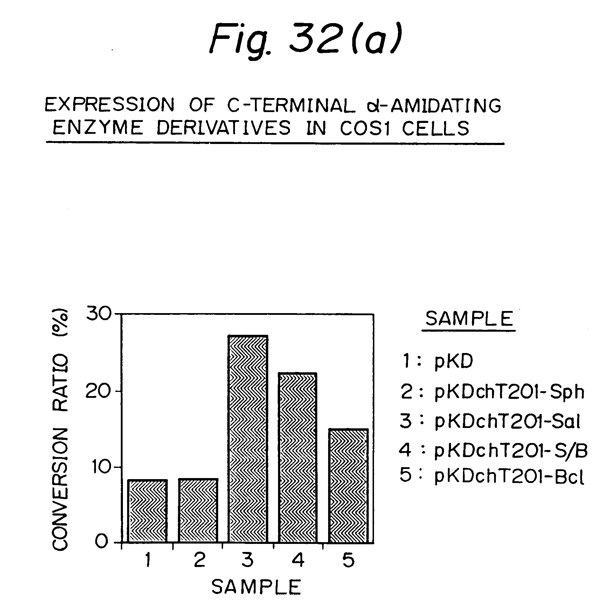

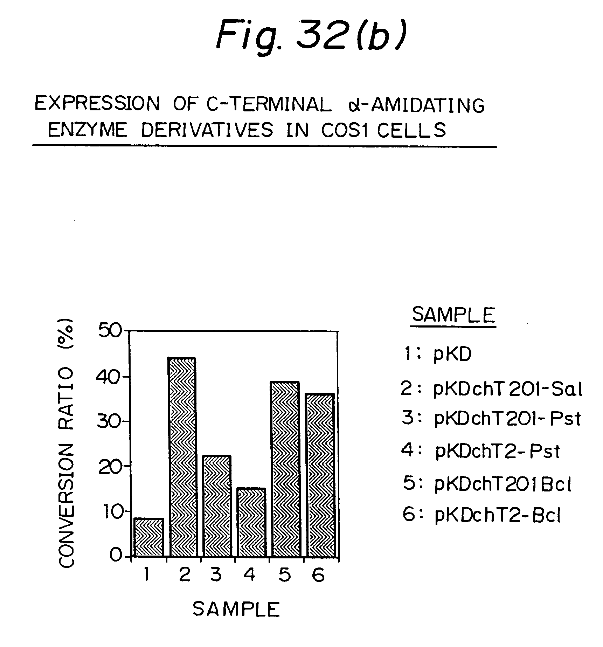

The C-terminal α-amidating enzyme activity provided by culturing the above-mentioned various transformants and transfectant is shown in Figs. 23, 32(a), and 32(b). In these Figures, the enzyme activity is expressed in the conversion ratio of substrate to amidated product.

As seen from the results shown in the Figures, although E. coli transformed with P_{L}chT201 MPF-Sph or P_{L}chT201 MFK-Sph, and COSl cells transfected with pKDch201-Sph did not provide a greater enzyme activity than that provided by the control strains, i.e., host E. coli or COSl cells transfected with pXD, all other transformants and transfectants did provide a greater enzyme activity than the corresponding control strain.

The cDNA inserts in the vectors P_{L}chT201 MFK-Sph, P_{L}chT02 MPF-Sph, and pKDchT201-Sph which did not provide a greater enzyme activity to that of the corresponding control, terminate at the 323th Ala in the amino acid sequence in Fig. 1, but the cDNA inserts in the vectors P_{L}chT201 MPF-Sal, P_{L}chT201 MFK-Sal, and pKDchT201-Sal, which did provide greater enzyme activity than the corresponding control, terminate at the 378th Lys in the amino acid sequence shown in Fig. 1 (also Fig. 3). Accordingly, it can be reasonably concluded that an amino acid sequence starting at the 37th Pro and ending at the 378th Lys in the amino acid sequence shown in Figs. 1 and 3 is sufficient to provide the desired enzyme activity. Note, the amino acid sequence of this region is identical in Fig. 1 and Fig. 3.

Accordingly, the present invention includes any chT201-type enzyme derivative having an amino acid sequence which starts at any amino acid selected from the first amino acid Met to the 37th Pro and terminates at any amino acid selected from the 378th Lys to the 974th Ser, in the amino acid sequence shown in Fig. 1. More specifically, the present chT201-type enzyme derivatives include those schematically shown in Fig. 33(a) and Fig. 34, except for those provided by P_{L}chT201 MFK-Sph, P_{L}chT201 MPF-Sph and pKDT201-Sph, in addition to native enzyme starting from the first Met and ending at the 974th Ser. Nevertheless, the present chT201-type enzyme derivatives should not be limited to the above-mentioned particular derivatives. Since all tested derivatives which terminate at the 378th Lys, the 434th Asp or the 857th Ile exhibit the desired enzyme activity, it can be reasonably believed that any derivative which terminates at any amino acid between the 378th Lys to the 974th Ser will provide the desired enzyme activity. These derivatives can be easily obtained by constructing an expression vector containing a shortened cDNA insert. The shortened cDNA fragment can be easily prepared by a conventional procedure, such as exonuclease digestion.

Similarly, the present invention includes any chT101-type enzyme derivatives having an amino acid sequence which starts at any amino acid selected from the first amino acid Met to the 37th Pro and terminates at any amino acid selected from the 378th Lys to the 866th Ser, in the amino acid sequence shown in Fig. 3. More specifically, the present chT101-type enzyme derivatives include those schematically shown in Fig. 33(b) and Fig. 34, in addition to native enzyme starting at the first Met and ending at the 866th Ser in Fig. 3. Nevertheless, the present chT101-type enzyme derivative should not be limited to the above-mentioned particular derivatives. Since all tested derivatives which terminate at 378th Lys (Same as chT201-type), the 461th Ala or the 750th Ile exhibit the desired enzyme activity, it can be reasonably believed that any derivative which terminates at any amino acid between the 379th Lys to the 866th Ser will provide the desired enzyme activity. These derivatives can be easily obtained by constructing an expression vector containing a shortened cDNA insert. The shortened cDNA fragment can be easily prepared by a conventional procedure, such as exonuclease digestion.

Moreover, the present enzyme derivatives can have an additional amino acid or amino acid sequence consisting of about up to ten amino acids, which is not present in the native sequence. In some cases, the additional amino acid or amino acid sequence is indispensably introduced by a multicloning site present in a vector used to construct an expression vector.

### (7) Use of the enzyme

The present enzyme products can be used for the preparation of α-amidated peptide. In this process, a substrate peptide having a glycine residue at the C-terminal thereof is incubated with one of the present enzyme products in an aqueous reaction medium, preferably in an aqueous buffer such as Tris-HCl, at a pH of about 6 to 7, and at a temperature of about 37°C, for a time sufficient to convert a substantial amount of the starting peptide to a corresponding C-terminal α-amidated peptide. Practically, the present enzymes can be used to produce physiologically active peptides having a C-terminal α-amide from precursor peptides having glycine at the C-terminal thereof, which precursor peptide may be produced by a recombinant DNA technique, chemical methods, or isolated from a natural source.

Although the present invention discloses in detail a process for the production of a C-terminal α-amidating enzyme using the cDNA in an E. coli host, and animal cells, the present cDNA can be used to express the target enzyme in another host, such as yeast cells.

### Examples

The present invention will now be further illustrated by, but is no means limited to, the following examples.

### Example 1. Preparation of poly(A) RNA from human thyroid grand

### (1) Preparation of total RNA

First, 14 g of human thyroid gland was used to prepare total RNA, and 10 ml/2 g the tissue of PC9 (phenol: CHCl₃: isoamylalcohol = 24: 24:10, saturated with 10 mM Tris-HCl, pH 9.0, 100 mM NaCl, 5 mM EDNA), and 10 ml/2 g the tissue of NETS solution (100 mM Tris-HCl, pH 9.0, 100 mM NaCl, 10 mM EDTA, 5% SDS) were added to the thyroid gland, and cells in the thyroid gland were disputed by a polytron. The disruptant was centrifuged at 3000 rpm for 20 minutes to obtain an aqueous layer, the residual phenol layer was mixed with the NETS solution, and the mixture was centrifuged as described above to obtain an aqueous layer. The aqueous layers were combined, to the combined aqueous layer was added the same volume of CHCl₃ , and the mixture was centrifuged at 3000 rpm for 20 minutes to obtain an aqueous solution; to which was added two volumes of ethanol, and the mixture then allowed to stand at -20°C overnight to form an ethanol precipitate.

The mixture was centrifuged at 3000 rpm and 4°C for 30 minutes to recover the precipitate, which was then washed with 80% ethanol and dried under vacuum. The dried precipitate was redissolved in 1 ml/g the tissue of guanidine thiocyanate solution (4.2 M guanidine thiocyanate, 0.1 M sodium acetate, 5 mM EDTA, pH 5.0), 4 ml of CsCl solution (5 M CsCl, 0.1 M sodium acetate, 5 mM EDTA, pH 5.0) was put into a SW40TI centrifugation tube, and the guanidine thiocyanate solution of the precipitate was overlaid on the CsCl solution in the tube. After centrifugation at 33,000 rpm and 25°C for 15 hours, the RNA precipitate at the bottom of the tube was washed with 80% ethanol, and the precipitate was dissolved in 500 µl of ETS solution (10 mM Tris-HCl, pH 7.5, 10 mM EDTA, 0.5% SDS). Then to the solution was added the same volume of the PC9, and after stirring, the mixture was centrifuged at 10,000 rpm for 5 minutes to obtain an aqueous layer. To the aqueous layer was added the same volume of ethyl ether, and after stirring, the mixture was centrifuged at 10,000 rpm for one minute to obtain an aqueous solution. Then to the solution was added two volumes of ethanol, the mixture was allowed to stand at -80°C for 30 minutes to form an ethanol precipitate, and was then centrifuged at 13,000 rpm and 4°C for 15 minutes to separate the precipitate. After removing the supernatant, the precipitate was washed with 80% ethanol and dried under vacuum. The dried precipitate was then redissolved in sterile distilled water, whereby about 7 mg of total RNA was obtained.

### (2) Preparation of poly (A) RNA

An oligo (dT) cellulose column was used to prepare poly(a) RNA. 0.5 g of oligo (dT) cellulose was packed to the column, which was washed with 10 ml of distilled water and 10 ml of 10 N NaOH, 5 mM EDTA, and the column was then thoroughly washed with distilled water. The column was equilibrated with 10 ml of a 1X loading buffer (20 mM Tris-HCl, pH 7.6, 0.5 M NaCl, 1 mM EDTA, 0.1% SDS), 7 mg of the total RNA in 13.5 ml of distilled water was heated at 65°C for 5 minutes, to the solution was added the same volume of 2x loading buffer, and the mixture then applied to the column. A flow-through fraction was heated again at 65°C for 5 minutes and again applied to the column. The column was washed with 4 ml of the loading buffer and 4 ml of 20 mM Tris-HCl (pH 7.6), 0.1 M NaCl, 1 mM EDTA and 0.1% SDS. The poly(A) RNA was then eluted with 4 ml of a elution buffer (10 mM Tris-HCl, pH 7.5, 1 mM EDTA, 0.05% SDS), and to the eluted fraction was added one tenth volume of 2 M sodium acetate (pH 5.0) and two volume of ethanol to recover the poly(A) RNA, whereby 144 µg of poly(A) RNA was obtained from 7 mg of the total RNA.

### Example 2. Preparation of λgtII cDNA library

From 5.6 µg of the poly(A) RNA prepared from a human thyroid gland as in Example 1, a double-stranded cDNA was prepared using an Amersham cDNA synthesis kit. Next, the double-stranded cDNA was cloned to the λgtII phage, to prepare a cDNA library according to the following procedure.

To 100 µl of the double-stranded cDNA solution were added 10 µl of 10% SDS and 10 µl of 0.25 M EDTA, followed by 120 µl of PC9. After stirring, the mixture was centrifuged at 10,000 rpm for 10 minutes to obtain an aqueous solution, to the aqueous solution, were added 120 µl of 4 M ammonium acetate and 480 µl of ethanol, the mixture was allowed to stand at -80°C overnight to form a precipitate, and then centrifuged at 13,000 rpm for 20 minutes to separate the precipitate, which was then washed with 80% ethanol and dried under vacuum. The precipitate was redissolved in 100 µl of 1x TE solution (10 mM Tris-HCl, pH 7.5, 1 mM EDTA), and an ethanol precipitation was carried out with 4 M ammonium acetate. The ethanol precipitate was dried and redissolved in 10 µl of distilled water, to the solution were added 4 µl of 5x EcoRI methylase buffer (400 mM Tris-HCl, pH 8.0, 40 mM EDTA, 8 mM DTT), 2 µl of 800 µM S-adenosylmethionine, 0.4 µl of EcoRI methylase (100: Takara Shuzo, Japan, and 3.6 µl of distilled water. The reaction mixture was incubated at 37°C for 10 minutes, and to the reaction mixture was added the same volume of PC9, and after stirring, the mixture was centrifuged at 13,000 rpm for 10 minutes to obtain an aqueous solution. After the addition of one tenth volume of 3 M sodium acetate and two volume of ethanol, the mixture was allowed to stand at -80°C for 30 minutes to form a precipitate. The mixture was then centrifuged to separate the precipitate, which was washed with 80% ethanol and dried. To the ethanol precipitate were added 1 µl of 10x ligation buffer (660 mM Tris-HCl, pH 7.6, 66 mM MgCl₂), 0.2 µl of 20 mM ATP, 15 µl of a 5'-phosphorylated EcoRI linker (12 mer, 0.1 µg/µl, Takara Shuzo, Japan), and 1 µl (5 units) of T4 DNA ligase (Takara Shuzo), and the reaction mixture was incubated at 15°C overnight. After the incubation, the mixture was heated at 65°C for 10 minutes, to inactivate the enzyme, and after the addition of 10 µl of 20 mM Tris-HCl (pH 7.5), 100 mM NaCl, and then 2 µl of EcoRI (20 units), the reaction mixture was incubated at 37°C for 5 hours. After the incubation, the reaction mixture was treated with PC9, and adjusted to a final volume of 30 µl by adding 1x TE. The mixture was applied to a Biogel A50 m column (0.5 x 25 cm) equilibrated with TES buffer (10 mM Tris-HCl, pH 7.5, 1 mM EDTA, 50 mM NaCl) and eluted with a TES buffer. A main peak fraction was obtained, and to the fraction was added 0.5 µg of phage λgtII which had been cleaved with EcoRI and then 5'-dephosphorylated (Pharmacia), and an ethanol precipitation was carried out. To the precipitate, were added 1.5 µl of 10x ligation buffer, 8 µl of distilled water, 1.5 µl of 0.1 M DTT, 1.5 µl of 10 mM ATP, and 1.5 µl of T4 DNA ligase (7.5 units), and the reaction mixture was incubated at 12°C overnight. Then 5 µl of the reaction mixture was subjected to in vitro λphage packaging, using a Giga pack gold from Strategene, and as a result, a cDNA library of a human thyroid gland origin consisting of about 500,000 clones was obtained.

Next, the cDNA library was amplified by the following procedure. E. coli VCS 257 (derived from supF; Maniatis, T. et al., Molecular Cloning, 1982, Cold Spring Harbor) was cultured in TB medium (10 g/l of Bactotrypron, 5 g/l of NaCl, pH 7.5) at 37°C overnight to prepare an inoculum. 40 ml of TB medium was inoculated with one hundredth of the inoculum, and culturing was carried out at 30°C overnight. The culture was centrifuged at 3000 rpm for 10 minutes, and after discarding the supernatant, to the precipitated cells was added 16 ml of 10 mM MgSO₄. Then, the cDNA library phage was added to the cell suspension, in an amount of 500,000 pfu (plaque forming units), and the whole was allowed to stand at 37°C for 20 minutes. To the culture was added 30 ml of NZY soft agar medium (10 g/l NZ amine, 5 g/l NaCl, 5 g/l yeast extract, 2 g/l MgSo₄ ·7H₂O and 10 g/l agar, pH 7.5), and the mixture was overlaid on an NZY medium containing 1.5% agar, in a petri dish (23 x 23 cm). After incubation at 37°C for 6 hours, 30 ml of an SM buffer (50 ml of 5.8 g/l NaCl, 2 g/l MgSo₄ ·7H₂O, 1 M Tris-HCl, pH 7.5; 2% gelatin 5 ml/l) was added to the petri dish, and the dish was allowed to stand at 4°C overnight. The SM buffer containing phages was recovered as a phage stock solution. This producure enabled an amplified cDNA library containing 4 x 10¹¹ pfu/ml phages to be prepared.

### Example 3. Isolation of λgtII phage containing cDNA coding for novel C-terminal α-amidating enzyme

### (1) Preparation of DNA probe

To isolate a phase clone containing a cDNA insert coding for a novel C-terminal α-amidating enzyme from the cDNA library derived from a human thyroid gland, a DNA probe was prepared by completely digesting plasmid pXA 799 containing a cDNA coding for an entire Xenopus laevis C-terminal α-amidating enzyme with restriction enzymes ApaI and DraI, and isolating an ApaI-DraI DNA fragment of about 2.8 kb containing an entire coding region, by agar gel electrophoresis. Next, the ApaI-DraI DNA fragment was radiolabeled with [α-³²P]dCTP, by nick translation.

### (2) Plaque hybridization

E. coli cells prepared by overnight culture and 150000 pfu of λgtII phage were mixed with 25 ml of a soft agar NZY medium, and the mixture was overlaid on a 23 x 23 cm NZY agar medium in a petri dish, and incubated at 37°C overnight to form plaques. Six petri dishes were prepared. A nitrocellulose filter (22 x 22 cm; Shleicher & Schnell) was put on the agar medium for 5 minutes, and then the nitrocellulose filter was put on an alkaline denaturation solution (0.1 N NaOH, 1.5 M NaCl) for 10 minutes, and thereafter, on a neutralizing buffer (0.5 M Tris-HCl, pH 7.5, 1.5 M NaCl) for 10 minutes. Next, the nitrocellulose filter was washed with 2x SSC solution (20 x SSC = 175.3 g/l NaCl, 88.2 g/l trisodium citrate), dried in air, and baked at 80°C under a reduced pressure for 120 minutes.

The nitrocellulose was then put into a polymer sack, to which 60 ml of a prehybridization solution [3x SSC, 20% formamide, 50 mM Tris-HCl (pH 7.5), 0.25 mM EDTA, 1x Denhardt's solution (1x Denhardt = 0.2 mg/ml each of albumin, polyvinyl pyrrolidone and Ficoll), 20 µg/ml salmon sperm DNA] was introduced thereto, and a prehybridization was carried out at 37°C for 3 hours. After discarding the prehybridization solution, 2,000,000 cpm/sack of the probe and 10 ml of the above-mentioned prehybridization solution were added to the sack, to carry out hybridization at 37°C overnight. Next, the filter was twice washed in 500 ml of 3x SSC containing 0.1% sodium dodecyl sulfate (SDS), at 37°C for 30 minutes, and then twice in 500 ml of 0.1x SSC containing 0.1% SDS, at 37°C for 40 minutes. The nitrocellulose was dried in air and subjected to autoradiography.

In this procedure, two phage clones hybridized with the ApaI-DraI DNA probe containing a cDNA coding for an Xenopus laevis C-terminal α-amidating enzyme were obtained from a cDNA library comprising 90,000 phages. These clones were designated as λgt11chT28 and λgt11chT40. Phage DNAs of the λgt11chT28 and λgt11chT40 were prepared according to T. Maniatis et al., Cold Spring Harbor Molecular Cloning 1982, and digestion of the phage DNA λgt11chT28 and λgt11chT40 with EcoRI provided a cDNA insert of about 0.7 kb and a cDNA insert of about 2.2 kb, respectively.

For each cDNA, a restriction enzyme cleavage map was prepared, cleaved with various restriction enzymes, and the resulting DNA fragments were subcloned in M13 phage to determine the nucleotide sequence, using a Takara DNA sequencing kit (Takara Shuzo, Japan) according to the dideoxy termination method (Sanger, F. et al., Pro. Natl. Acad. Sci. V.S.A. 34, 5463-5467, 1977). As a result, it was found that the λgt11ch28 contained a cDNA insert coding for a protein similar to a central portion of a protein encoded by the pXA799; and the λgt11ch40 contained a cDNA insert coding for a protein similar to a C-terminal half of protein encoded by the pXA 799 (See Fig. 7). Therefore, it was assumed that both the λgt11chT28 and λgt11ch40 partially encode a C-terminal α-amidating enzyme of human thyroid origin.

Accordingly, to obtain a phage clone which contains a cDNA insert coding for an entire enzyme, the same cDNA library was screened. A desired clone could not be isolated, however, and therefore another cDNA library was prepared as follows.

### Example 4. Preparation of λgt10 cDNA library

4 µg of the poly(A) RNA prepared in Example 1 was used to prepare double-stranded cDNA using a cDNA synthesis kit (Pharmacia). The resulting double-stranded cDNA and 3 µg of λgt10 phage (Strategene) were ligated, using a T4 DNA ligase, at 12°C overnight, and subjected to in vitro packaging. As a result, a λgt10 cDNA library comprising about 2 x 10⁶ clones was obtained.

### Example 5. Isolation of phage clones λgt10chT2, λgt10chT201 and λgt10chT101 containing cDNA coding for desired enzyme

### (1) Preparation of DNA probes

To isolate phage clones containing a cDNA insert coding for an entire C-terminal α-amidating enzyme of human thyroid gland origin, in addition to the ApaI-DraI DNA probe prepared in Example 3 (1), an additional DNA probe was prepared by cleaving the above-prepared λgt11chT28 with EcoRI to obtain a 0.7 kb EcoRI DNA fragment, which was then radiolabeled with [α-³²P]dCTP by nick translation.

### (2) Hybridization

About 280,000 clones of the λgt10 cDNA library were plated on 23 x 23 cm petri dishes, using E. coli C600 hfℓ⁻ as an indicator strain as described in Example 3. (2).

After an overnight culture at 37°C, the formed plaques were transferred to a nitrocellulose filter, which was then subjected to alkaline denaturation, neutralization, air drying at 80°C under a reduced pressure, and baking for 90 minutes, as described in Example 3. (2). The nitrocellulose filter was put into a polymer sack, to which 50 ml of the above-mentioned prehybridization solution additionally containing 20% formamide was introduced. The sack was incubated at 37°C for 3 hours, and after discarding the solution 10,000,000 cpm of the ApaI-DraI DNA probe and 10 ml of the prehybridization solution containing 20% formamide were introduced into the sack, which was then incubated at 37°C overnight. Next, the filter was twice washed is 500 ml of 3 x SSC solution containing 0.1% SDS, and then twice washed with a 0.1 x SSC solution containing 0.1% SDS, at 37°C. After air drying, the filter was subjected to autoradiography, and as a results one positive clone which hybridized with the ApaI-DraI probe derived from pXA 799 was obtained, and was designated λgt10chT2.

The same procedure as described above was repeated, except that 340,000 clones of the λgt10 cDNA library and 10,000,000 cpm of the EcoRI DNA probe derived from λgt11chT28 were used. The prehybridization and hybridization were carried out as described above, except that the prehybridization solution contained 50% formamide, and as a result, two positive clones hybridized with a 0.7 kb EcoRI DNA probe from λgt11chT28 were obtained, and were designated λgt10chT201 and λgt10chT202.

Phage DNAs were prepared for three clones thus obtained by a conventional procedure, and to test the length of cDNA insert, each phage DNA was digested with EcoRI. As a result, the λgt10chT2 provided two DNA fragments of about 2.4 kb and 0.4 kb, showing that the 2.8 kb cDNA had been inserted. On the other hand, the λgt10chT201 provided three DNA fragment of about 2.2 kb, 0.8 kb, and 0.7 kb, respectively, showing that the 3.7 kb cDNA had been inserted.

### (3) Determination of nucleotide sequences of cDNAs inserted in λgt10chT2 and λgt10chT201

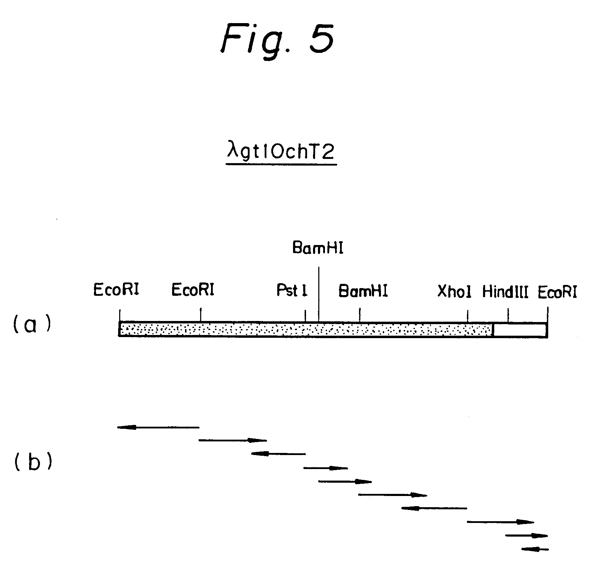

The λgt10chT201 and λgt10chT2 phage DNAs were digested with various restriction enzymes, and the resulting fragments were separated by agar gel electrophoresis to prepare restriction enzyme cleavage maps of cDNA regions. The results are shown in Fig. 4(a) and Fig. 5(a). Next, to determine the nucleotide sequence of each cDNA insert, the DNA fragments were subcloned in M13 phage, and the nucleotide sequence was determined by using a Takara DNA sequencing kit (Takara Shuzo, Japan) and a Sequanase^{(TM)} DNA sequencing kit (United States Biochemical Corporation), according to the dideoxy termination method. The strategies used for the sequencing are shown in Figs. 4(b) and 5(b). The Nucleotide sequence of cDNA in the phage clone λgt10chT201 (abbreviated as chT201 cDNA) is shown in Fig. 1; and the nucleotide sequence of cDNA in the λgt10chT2 (abbreviated as chT2 cDNA) is shown in Fig. 2.

As seen from Fig. 1, the chT201 cDNA has a length of about 3.7 kb, and is a full length cDNA including a 5'-terminal non-coding region, a long reading frame, and a 3'-terminal non-coding region. On the other hand, the chT2 cDNA appears to lack the 5'-terminal region of a full length cDNA. In a comparison of the nucleotide sequences of the chT201 cDNA and chT2 cDNA, the chT201 cDNA contains 321 extra nucleotides coding for 107 amino acids, which are absent from the chT2 cDNA. The other portions are roughly identical for both the chT201 cDNA and the chT2 cDNA. In a comparison of the nucleotide sequence of the cDNA in pXA 799 derived from Xenopus laevis, as shown in Fig. 7, the chT2 cDNA is similar to the cDNA in PXA 799. On the other hand, the chT201 cDNA contains 321 nucleotides coding for 107 amino acids, which are absent from the cDNA in pXA 799.

This suggests that there are two types of C-terminal α-amidating enzymes derived from human thyroid gland.

### (4) Isolation of λgtch10T101 phage clone

As discussed above, the comparison of chT201 cDNA with chT2 cDNA suggested the presence of two types of C-terminal α-amidating enzymes of human thyroid gland origin, i.e., an enzyme encoded by chT201 cDNA having 321 additional nucleotides, and an enzyme encoded by chT2 cDNA not having the 321 nucleotides.

The chT2 cDNA, however, lacks the 5-terminal portion of a postulated full length cDNA, and accordingly, to obtain a chT2-type full length cDNA, the λgt10 cDNA library was again screened.

A DNA probe was prepared by cleaving the chT201 cDNA with SmaI and XhoI to obtain a 380 bp DNA fragment comprising a 5'-noncoding region and 5'-terminal side coding region, and labeling the DNA fragment with [α-³²P]dCTP by nick translation. Plaque hybridization was carried out according to the procedure described in Example 5. (2), using 1,280,000 clones of the λgt10 cDNA library, the 380 bp DNA probe, and the prehybridization solution containing 50% formamide, to obtain one positive clone. The clone was designated as λgt10chT101. Phage DNA of λgt10ch101 was prepared by a conventional procedure, and the length of the cDNA insert in the λg10chT101 (abbreviated as chT101 cDNA) was determined by cleaving the phage DNA. As a result, the cleavage provided three cDNA fragments of 0.7 kb, 0.9 kb, and 2.6 kb, showing that the λgt10chT101 phage clone contains the 4.2 kb cDNA insert.

### (5) Determination of nucleotide sequence of cDNA insert in λgt10chT101

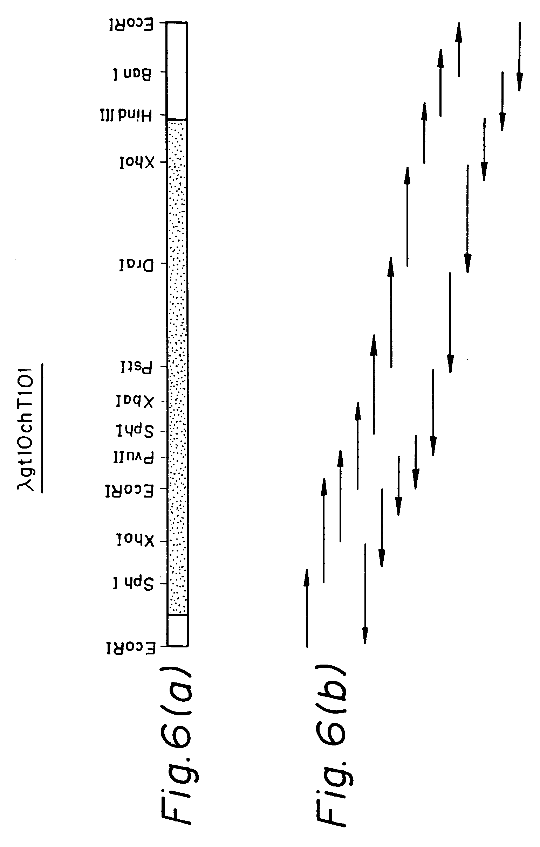

The λgt10chT101 phage DNA was cleaved with various restriction enzymes, and the resulting fragments were separated by agar gel electrophoresis to prepare a restriction enzyme cleavage map of chT101 cDNA. The result is shown in Fig. 6(a). Next the DNA fragments were subcloned in M13 phage, and a nucleotide sequence was determined using a Sequanase^{(TM)} DNA sequencing kit according to the dideoxy termination method. The strategy for the sequencing is shown in Fig. 6(b). The nucleotide sequence of chT101 cDNA, and a corresponding amino acid sequence, are shown in Fig. 3. Although the chT101 cDNA consists of three EcoRI fragments of 2.6 kg, 0.9 kb, and 0.9 kb, since the 0.9 kb cDNA fragment does not encode a C-terminal α-amidating enzyme, the nucleotide sequence of the 0.9 kb cDNA fragment is excluded from Fig. 3. As seen from a comparison of the nucleotide sequences shown in Figs. 2 and 3, both the chT101 cDNA and chT2 cDNA encode the same enzyme, although the chT101 cDNA encodes the entire enzyme and the chT2 cDNA encodes the same enzyme but lacking its N-terminal portion.

Figure 7 shows the relationship between pXA 799 cDNA, chT210 cDNA, chT2 cDNA, chT101 cDNA, chT40 cDNA, and chT28 cDNA.

### Example 6. Construction of E.coli vector expressing chT201-type protein derivatives, P_{L}chT201 MPF-Sph, P_{L}chT201 MPF-S/B, P_{L}chT201 MPF-Sal and P_{L}chT201 MPF-Bcl

To express protein derivatives encoded within the ch201 cDNA (nucleotides 140-3061) in E. coli, expression vectors P_{L}chT201 MPF-Sph, P_{L}chT201 MPF-S/B, P_{L}ch201 MPF-SalI, and P_{L}chT201 MPF-Bcl, as well as E. coli W3110 transformed with the expression vector, were prepared as follows.

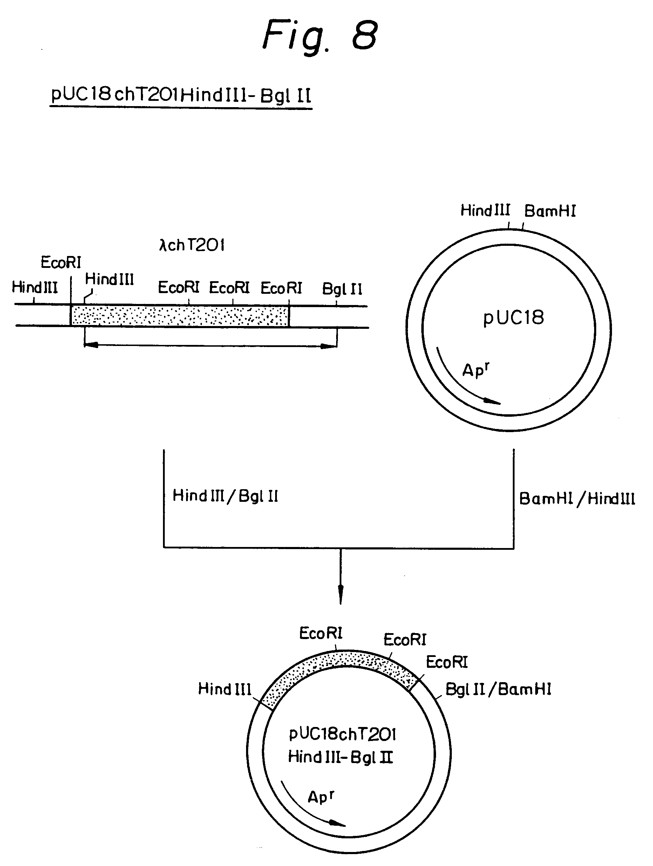

### (1) Construction of pUC18chT201 HindIII-BglII (Fig. 8)

As shown in Fig. 8, the λchT201 phage DNA was cleaved with HindIII and BglII to isolate aDNA fragment of about 5 kb comprising an entire coding region of the chT201 cDNA and a part of the λgt10 phage. On the other hand, pUC18 was cleaved with HindIII and Bam HI. Both DNA fragments were ligated to construct an intermediate plasmid pUC18chT201 HindIII-BglII, which was used to transform E. coli, and the transformant was designated as E. coli SBM302, and deposited with the FRI under the Budapest Treaty as FERM BP-2237, on January 11, 1989.

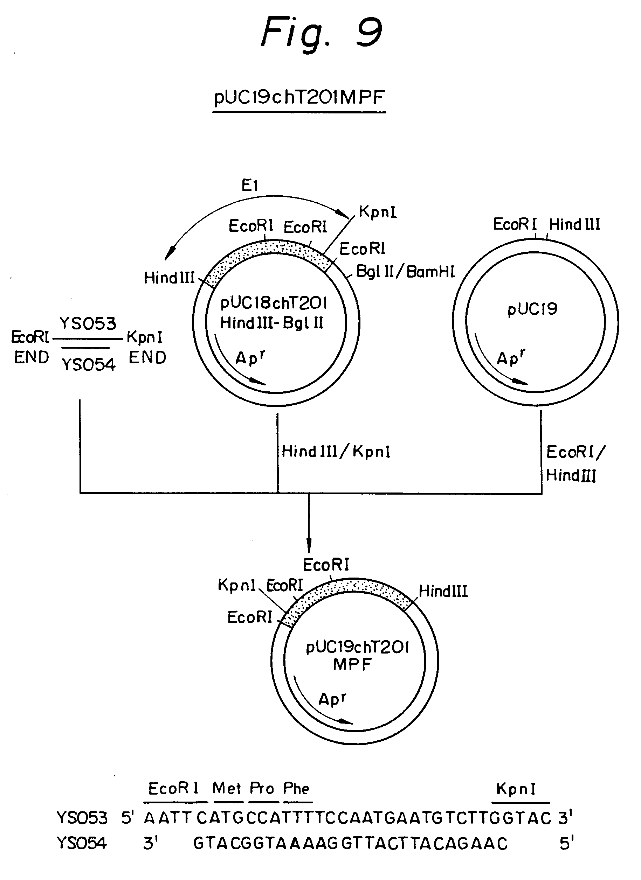

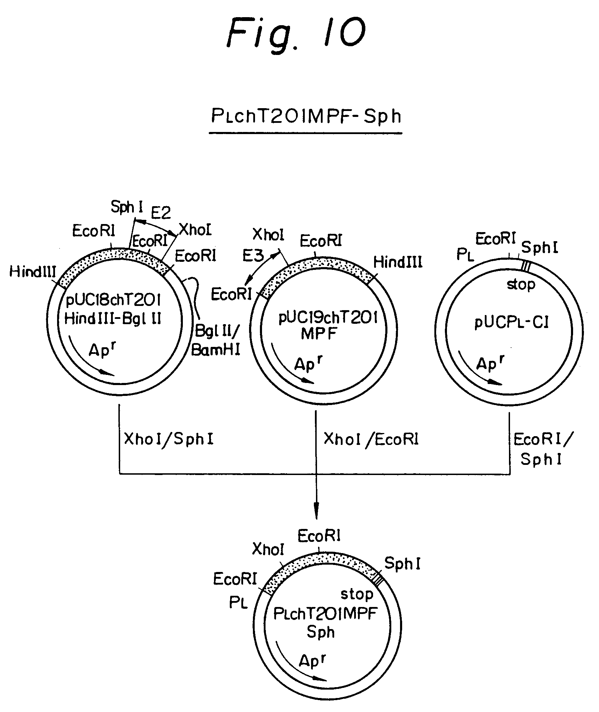

### (2) Construction of P_{L}ch201 MPF-Sph (Figs. 9 and 10)

There are two possible processing sites in an N-terminal region of a protein encoded by the chT201 cDNA, i.e., the bond between Arg(30)-Pro(31) and the bond between Arg(36)-Pro(37). To express protein derivatives starting with the Pro(37), a plasmid pUC19chT201 MPF was constructed.

As seen in Fig. 9, the plasmid pUC18chT201 HindIII-BglII was cleaved with KpnI and HindIII, to isolate a KpnI-HindIII DNA fragment (E1), and a DNA fragment YSO53 was synthesized having a 5'-EcoRI end, a translation start codon ATG, codons for the 37th Pro to the 43th Leu and a 3'-KpnI end, in this order. Further, a complementary DNA fragment YSO54 was synthesized. The DNA fragments YSO53 and YSO54 were 5'-phosphorylated using a T4 polynucleotide kinase and ATP, and annealed at 65°C for 10 minutes to prepare the double-stranded DNA fragment shown in Fig. 9. On the other hand, pUC19 was cleaved with EcoRI and HindIII. The synthetic DNA fragment, the E1 fragment, and the linearized pUC19 were ligated and used to transform E. coli W3110, to obtain a plasmid P_{L}chT201 MPF-Sph and corresponding transformant E. coli W3110/P_{L}chT201 MPF-Sph.

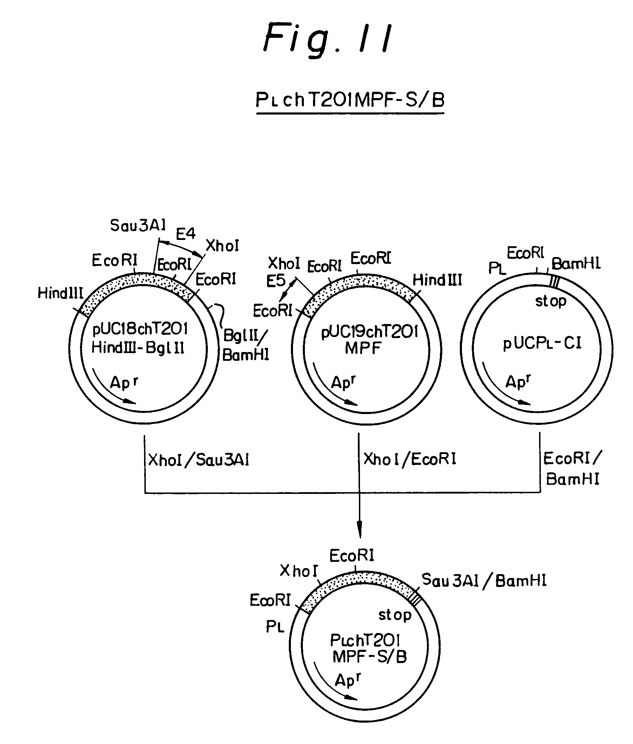

### (4) Construction of P_{L}chT201 MPF-S/B (Fig. 11)

As shown in Fig. 11, the plasmid pUC18chT201 HindIII-BglII was cleaved with XhoI and Sau3A1 (cleaving cDNA at 1439th position) to obtain a DNA fragment E4, and the plasmid pUC19chT201 MPF was cleaved with EcoRI and XhoI to obtain a DNA fragment E5. The plasmid pUCP_{L}-CI was cleaved with EcoRI and BamHI. Next, the DNA fragments E4 and E5, and the linearized pUCP_{L}-CI were ligated and used to transform E. coli W3110 to obtain a plasmid P_{L}chT201 MPF-S/B and corresponding transformant E. coli W3110/P_{L}chT201 MPF-S/B.

### (5) Construction of P_{L}chT201 MPF-Sal

To construct P_{L}chT201 MPF-Sal, first plasmids pUC19chT201-EcoRI 0.7 and pUC18-Sal were constructed.

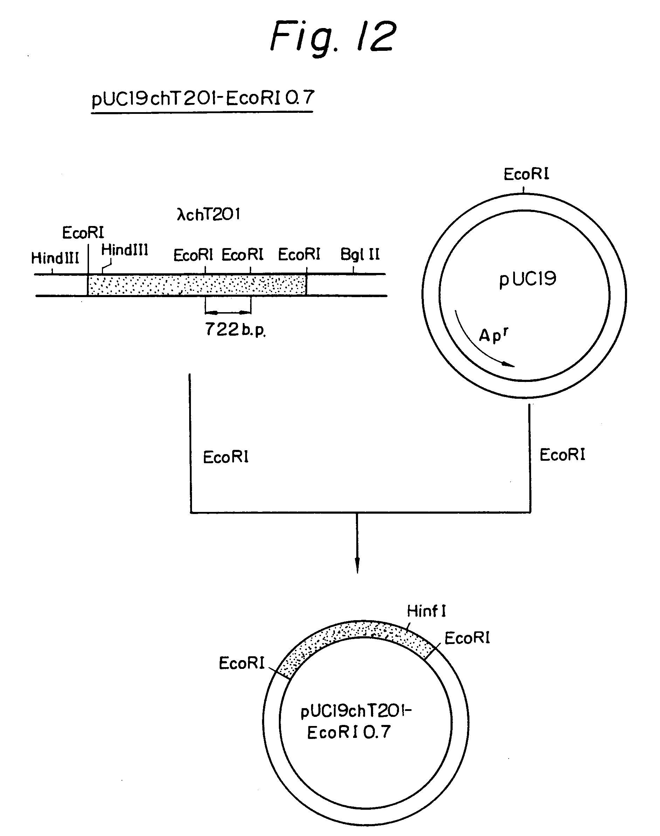

### (a) Construction of pUC19chT201-EcoRI 0.7 (Fig. 12)

As shown in Fig. 12, the λchT201 phage DNA was cleaved with EcoRI to obtain a 0.73 kb EcoRI DNA fragment. On the other hand, pUC19 was cleaved with EcoRI. The 0.73 kb EcoRI DNA fragment and the linearlized pUC19 were ligated an dused to transform E. coli JM109, to obtain a plasmid pUC19chT201-EcoRI 0.7 and corresponding transformant E. coli JM109/pUC19chT201-EcoRI 0.7.

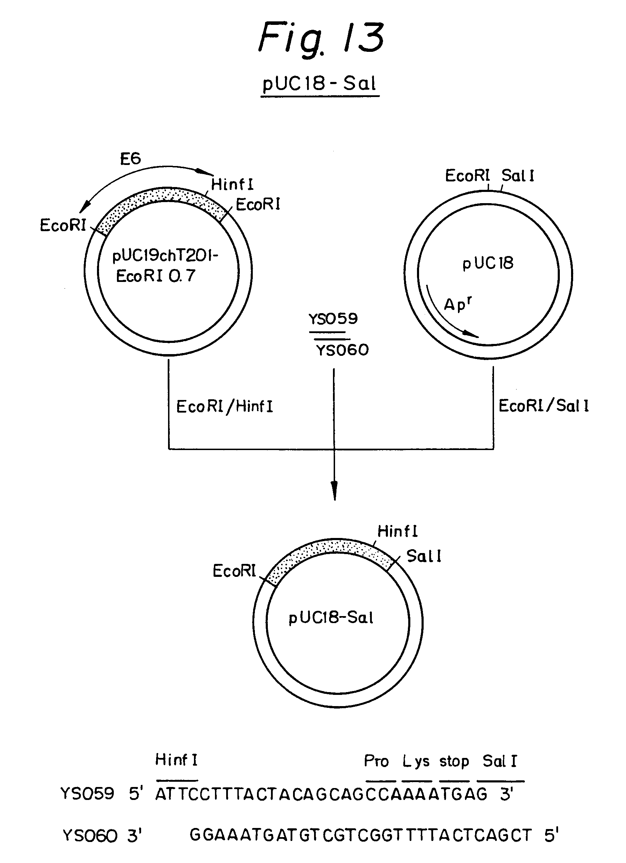

### (b) Construction of pUC18-Sal (Fig. 13)

As shown in Fig. 13, the plasmid pUC19chT201-EcoRI 0.7 was cleaved with EcoRI and HinfI (cleaving cDNA at 1250 position) to obtain a DNA fragment E6. To introduce a translation stop codon TGA immediately downstream of the 378th Lys in the chT201cDNA, a DNA fragment YSO59 having an Hinf cohesive end at its 5'-end, and a DNA fragment YSO60 complementary to the YSO59 and having a SalI cohesive end at its 5'-end, were synthesized. The YSO59 and YSO60 were 5'-phosphorylated using a T4 polynucleotide kinase and ATP, and annealed at 65°C for 10 minutes. On the other hand, pUC18 was cleaved with EcoRI and SalI. Next the annealed YSO59 and YSO60, the E6 DNA fragment, and the linearlized pUC18 were ligated and used to transform E. coli JM109, to obtain a plasmid pUC18-Sal and corresponding transformant E. coli JM109/pUC18-Sal.

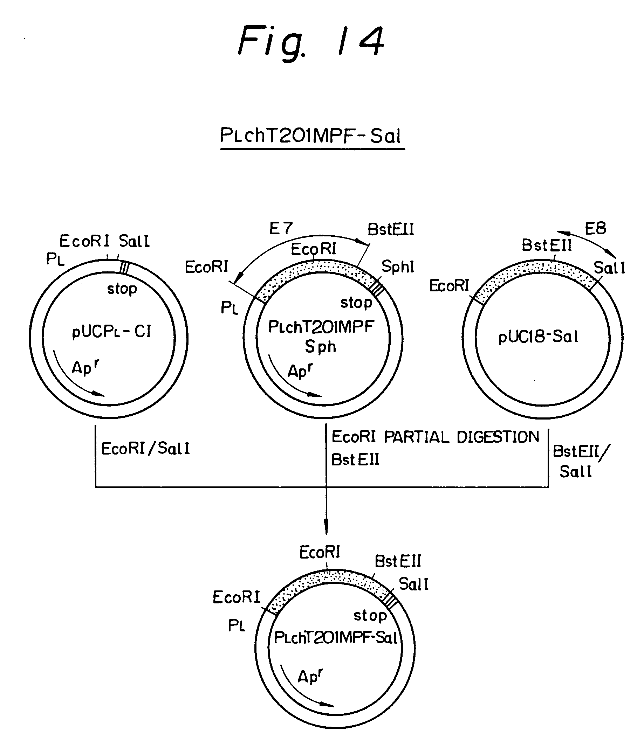

### (c) Construction of P_{L}chT201 MPF-Sal (Fig. 14)

As shown in Fig. 14, P_{L}chT201 MPF-Sph was cleaved with BstEII and then partially digested with EcoRI, to obtain an EcoRI-BstEII DNA fragment E7. On the other hand, pUC18-Sal was cleaved with SalI and BstEII to obtain a BstEII-SalI DNA fragment E8. Further, pUCP_{L}-CI was cleaved with EcoRI and SalI. The DNA fragments E7 and E8, and the linearized pUC18 were ligated and used to transform E. coli W3110, whereby a plasmid P_{L}chT201 MPF-Sal and corresponding transformant E. coli W3110/P_{L}chT201 were obtained.

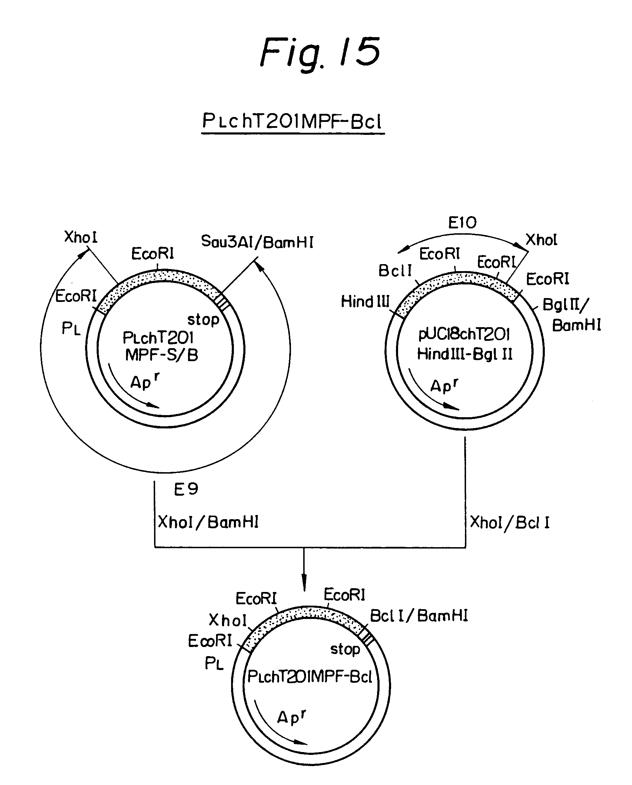

### (6) Construction of P_{L}chT201 MPF-Bcl (Fig. 15)

As shown in Fig. 15, P_{L}chT201 MFF-S/B was cleaved with XhoI (cleaving cDNA at 421 position) and BamHI to obtain an XhoI-BamHI DNA fragment E9. On the other hand, pUC18chT201 HindIII-BglII was cleaved with XhoI and BclI (cleaving cDNA at 2707 position) to obtain an XhoI-BclI DNA fragment E10. The DNA fragments E9 and E10 were ligated and used to transformant E. coli W3110, whereby a plasmid P_{L}chT201 MPF-Bcl and corresponding transformant E. coli W3110/P_{L}chT201 MPF-Bcl were obtained.

### Example 7. Construction of E. coli vector expressing chT201-type protein derivatives, P_{L}chT201 MFK-Sph, P_{L}chT201 MFK-S/B, P_{L}chT201 MFK Sal and PLchT201 MFK-Bcl

To express protein derivatives encoded within chT201 cDNA (nucleotide 140-3061) in E. coli, expression vectors P_{L}chT201 MFK-Sal, P_{L}chT201 MFK-S/B, P_{L}chT201 MFK-Sal and P_{L}ch201 MFK-Bcl, and corresponding E. coli W3110 transformants, were prepared as follows.

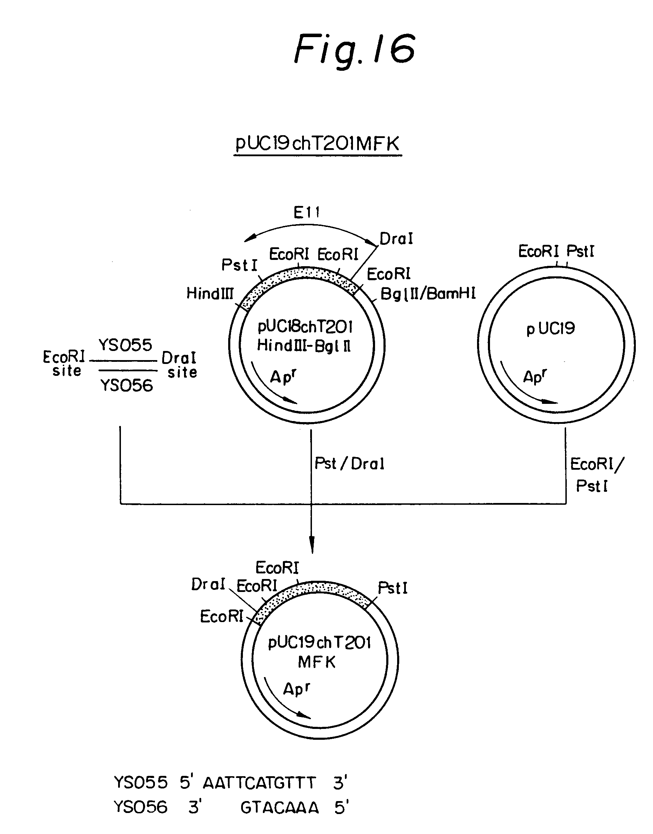

### (1) Construction of pUC19chT201 MFK (Fig. 16)

To express protein derivatives starting with the 31th Phe, a first plasmid pUC19chT201 MFK was constructed. For this purpose, as shown in Fig. 16, a DNA fragment YSO55 having a 5'-EcoRI end, a translation start codon ATG, a codon for the 31th Phe and 3'-DraI blunt end in this order, as well as a DNA fragment YSO56 complementary to the YSO55, were synthesized. These synthetic DNA fragments were 5'-phosphorylated using a T4 polynucleotide kinase and ATP, and annealed at 65°C for 10 minutes to prepare a double-stranded DNA fragment shown in Fig. 16. The plasmid pUC18chT201 HindIII-BglII was cleaved with PstI (cleaving cDNA at 1844 position) and DraI (cleaving cDNA at 233 position) to obtain a PstI-DraI DNA fragment E11. The synthetic DNA fragment, the DNA fragment E11, and the linearized pUC19 were ligated and used to transform E. coli JM109, whereby a plasmid pUC19ch201 MFK and corresponding transformant E. coli JM109/pUC19chT201 MFK were obtained.

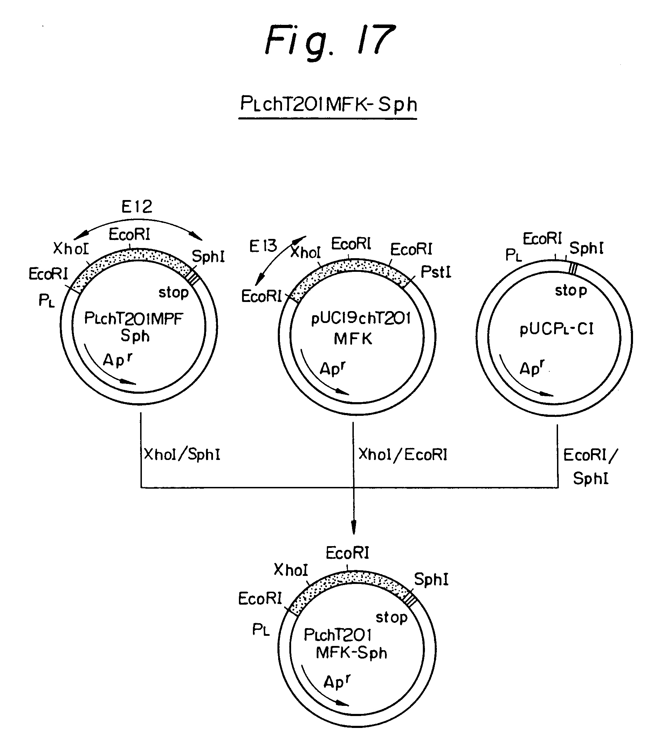

### (2) Construction of P_{L}chT201 MFK-Sph (Fig. 17)

As shown in Fig. 17, P_{L}chT201 MPF-Sph was cleaved with SphI and XhoI to obtain an SphI-XhoI DNA fragment E12. The plasmid pUC19chT201 MFK was cleaved with EcoRI and XhoI to obtain an EcoRI-XhoI DNA fragment E13, and pUCP_{L} CI was cleaved with EcoRI and SphI. Next, the DNA fragments E12 and E13, and the linearized pUCP_{L} CI, were ligated and used to transform E. coli W3110, whereby a plasmid P_{L}chT201 MFK-Sph and corresponding transformant E. coli W3110/P_{L}chT201 MFK-Sph were obtained.

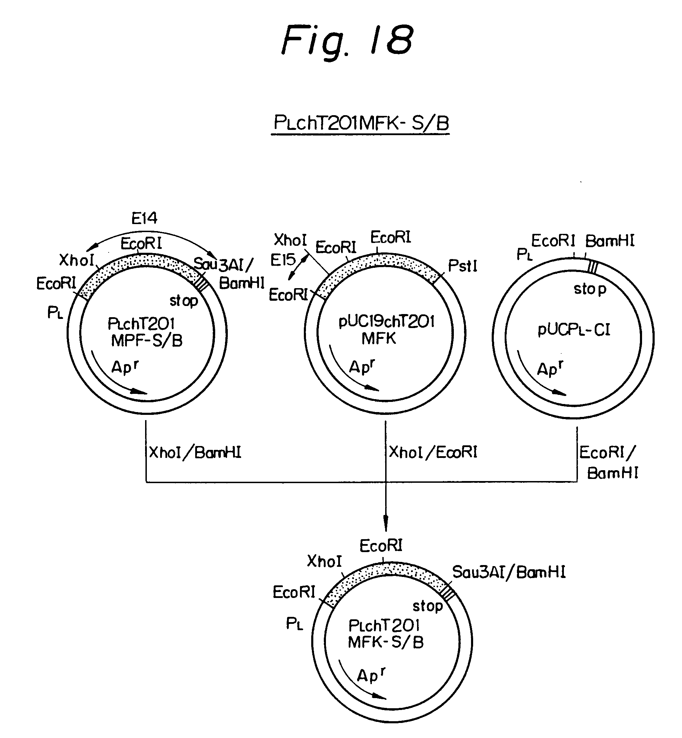

### (3) Construction of P_{L}chT201 MFK-S/B (Fig. 18)

As shown in Fig. 18, P_{L}chT201 MPF-S/B was cleaved with XhoI and BamHI to obtain an XhoI-BamHI DNA fragment E14. The plasmid pUC19chT201 MFK was cleaved with EcoRI and XhoI to obtain an EcoRI-XhoI DNA fragment E15. On the other hand, pUCP_{L} CI was cleaved with EcoRI and BamHI. Next, the DNA fragments E14 and E15, and the linearized pUCP_{L} CI, were ligated and used to transform E. coli W3110, whereby a plasmid P_{L}chT201 MFK-S/B and corresponding transformant E. coli W3110/P_{L}chT201 MFK-S/B were obtained.

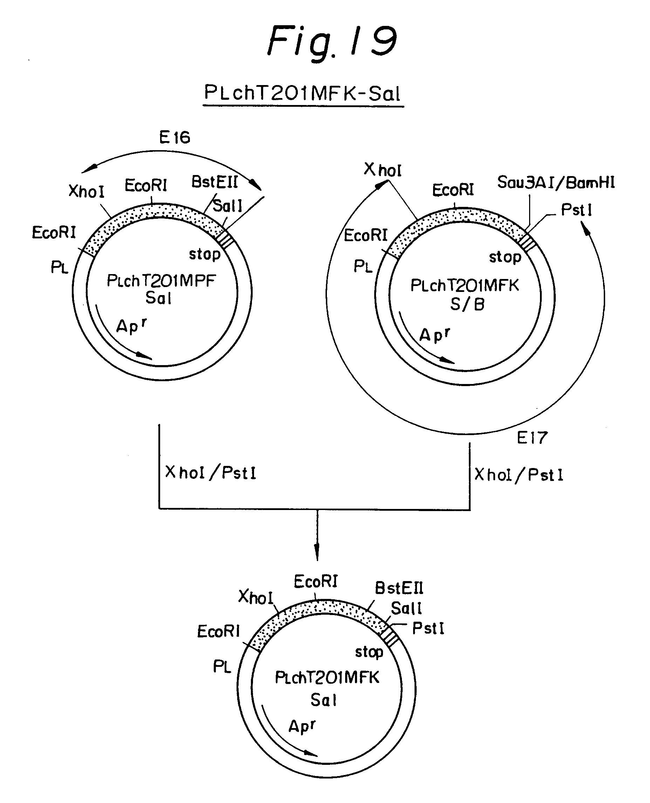

### (4) Construction of P_{L}chT201 MFK-Sal (Fig. 19)

As shown in Fig. 19, P_{L}chT201 MPF-Sal was cleaved with XhoI and PstI to obtain an XhoI-PstI DNA fragment E16. On the other hand, P_{L}chT201 MFK-S/B were cleaved with XhoI and PstI to obtain an XhoI-PstI DNA fragment E17. The DNA fragments E16 and E17 were ligated and used to transform E. coli W3110, whereby a plasmid P_{L}chT201 MFK-Sal and corresponding transformant E. coli W3110/P_{L}chT201 MFK-Sal were obtained.

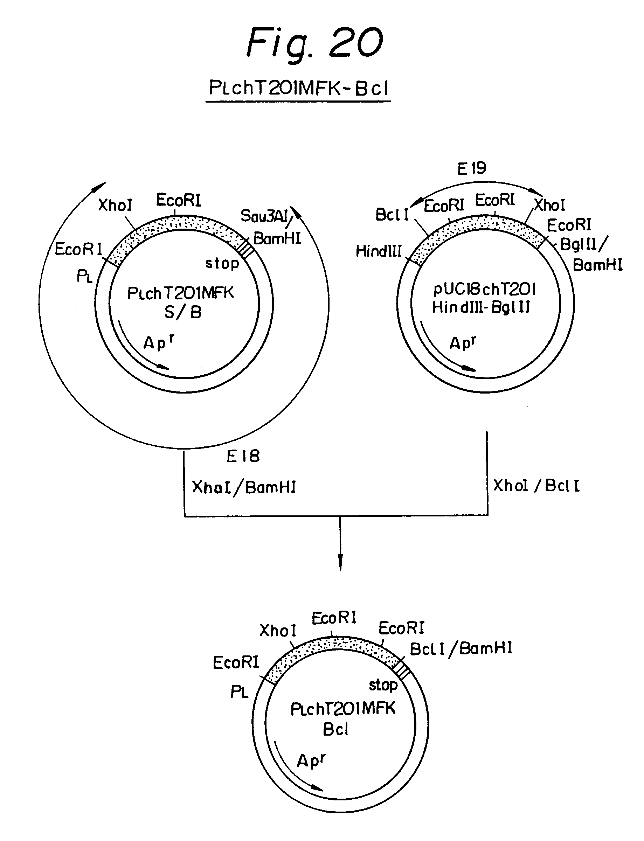

### (5) Construction of P_{L}chT201 MFK-Bcl (Fig. 20)

As shown in Fig. 20, P_{L}chT20 MPF-S/B was cleaved with XhoI and BamHI to obtain an XhoI-BamHI DNA fragment E18. On the other hand, pUC18chT201 HindIII-BglII was cleaved with XhoI and BclI to obtain an XhoI-BclI DNA fragment E19. Next, the DNA fragments E18 and E19 were ligated and used to transform E. coli W3110, whereby a plasmid P_{L}chT201 MFK-Bcl and corresponding transformant E. coli W3110/P_{L}chT201 MFK-Bcl were obtained.

### Example 8. Construction of E. coli vectors expressing chT101-type protein derivatives, P_{L}chT2 MPF-Pst, P_{L}chT2 MFK-Pst, P_{L}chT2 MPF-Bcl and P_{L}chT2 MFK-Bcl

Although the chT2 cDNA lacks the N-terminal coding region, the lacking region of chT2 cDNA can be supplemented with a corresponding region of chT201 cDNA, to construct a cDNA identical with the chT101 cDNA. Accordingly, to construct E. coli vectors expressing chT101-type C-terminal α-amidating enzyme derivatives, the chT2 cDNA and chT201 cDNA were used.

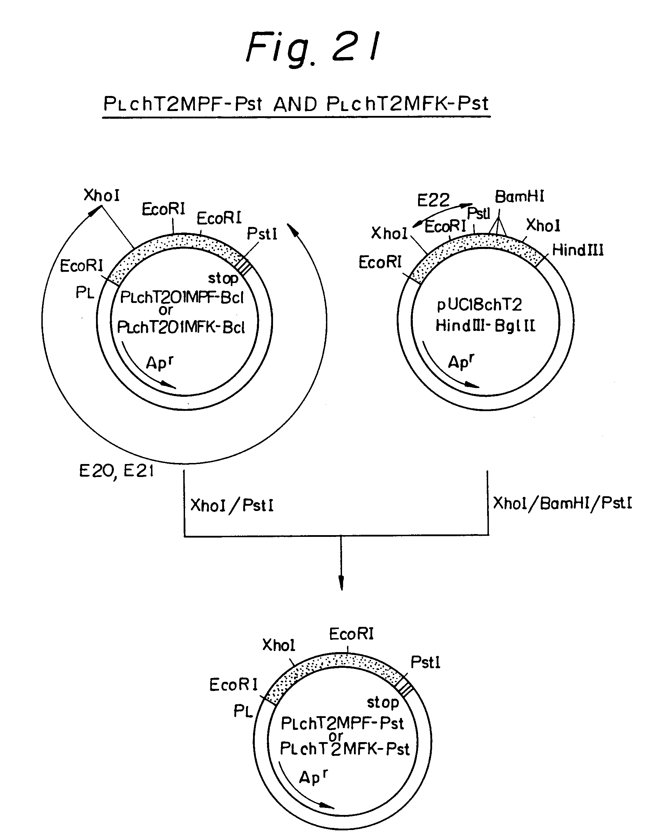

### (1) Construction of P_{L}chT2 MPF-Pst and P_{L}chT2 MFK-Pst (Fig. 21)

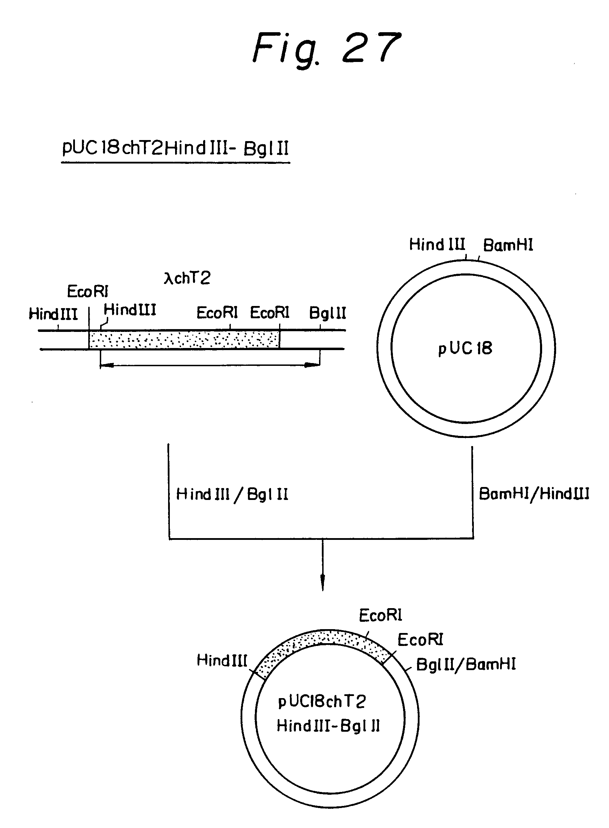

As shown in Fig. 21, P_{L}chT201 MPF-Bcl was cleaved with XhoI and PstI to obtain an XhoI-PstI DNA fragment E20. On the other hand, pUC18chT2 HindIII-BglII constructed in Example 10. (4) (see, Fig. 27) was cleaved with XhoI, BamHI and PstI to obtain an XhoI-PstI DNA fragment E22. Next, the DNA fragments E20 and E22 were ligated and used to transform E. coli W3110, whereby a plasmid P_{L}chT2 MPF-Pst and corresponding transformant E. coli W3110/P_{L}ch2 MFK-Pst were obtained.

The same procedure as described above was repeated except that P_{L}chT201 MFK-Bcl was used in place of P_{L}chT201 MPF-Bcl to obtain a plasmid P_{L}chT2 MFK-Pst and corresponding transformant E. coli W3110/P_{L}ch MFK-Pst.

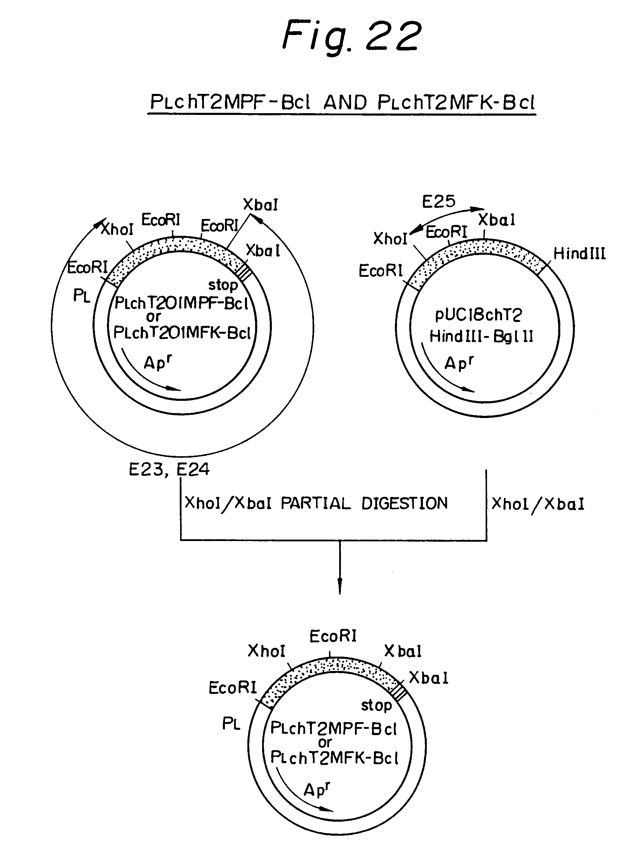

### (2) Construction of P_{L}chT2 MPF-Bcl and P_{L}chT2 MFK-Bcl (Fig. 22)

As shown in Fig. 22, P_{L}chT201 MPF-Bcl was cleaved with XhoI and then partially digested with XbaI to obtain an XhoI-XbaI DNA fragment E23. On the other hand, the pUC18chT2 HindIII-BglI constructed in Example 10. (4) (see Fig. 27) was cleaved with XhoI and XbaI to obtain an XhoI-XbaI DNA fragment E25. Next, the DNA fragments E23 and E25 were ligated and used to transform E. coli W3110, whereby a plasmid P_{L}chT2 MPF-Bcl and corresponding transformant E. coli W3110/P_{L}chT2 MPF-Bcl were obtained.

The procedure as described above was repeated except that P_{L}chT2 MFK-Bcl was used to in place of P_{L}chT2 MPF-Bcl, to obtain a plasmid P_{L}chT2 MFK-Bcl and corresponding transformant E. coli W3110/P_{L}chT2 MFK-Bcl.

### Example 9. C-terminal α-amidating activity provided by transformed E. coli

E. coli transformants prepared as above, W3110/P_{L}chT 201 MPF-Sph, W3110/P_{L}chT201 MPF-Sal, W3110/P_{L}chT201 MPF-S/B, W3110/P_{L}chT201 MPF-Bcl, W3110/P_{L}chT201 MFK-Sph, W3110/P_{L}chT201 MFK-Sal, W3110/P_{L}chT201 MFK-S/B, W3110/P_{L}chT201 MFK-Bcl, W3110/P_{L}chT2 MPF-Pst, W3110/P_{L}chT2 MFK-Pst, W3110/P_{L}chT2 MPF-Bcl, and W3110/P_{L}chT2 MFK-Bcl were separately cultured in a Super broth supplemented with 50 µg/ml ampicillin, at 32°C overnight. As a control E. coli W3110 (not transformed) was cultured in Super broth not containing ampicillin, at 32°C overnight. Note, the Super broth medium was prepared by dissolving 24 g of a yeast extract, 12 g of trypton, 5 ml of glycerol, and 100 ml of a 1 M phosphate buffer (pH 7.6) in water, to a total volume of 1 liter. Next, the culture was inoculated to 5 ml of a fresh medium having the same composition to 0.01 OD/ml at 660 nm, and culturing was carried out at 32°C until the absorbance at 660 nm become 0.3 OD/ml. At this time, the temperature was changed from 32°C to 42°C, and culturing was continued for further 3 hours. The cultured broth having an absorbance of 5 OD at 660 nm was centrifuged at 13,000 rpm for 2 minutes, to collect cells, the supernatant was discorded, the cells were suspended in 300 µl of PBS(-) (0.8% NaCl, 0.02% KCl, 0.15% Na₂HPO₄ and KH₂PO₄) containing 0.1% Triton, and the suspension was subjected to sonication to disrupt the cells. The disruptant was centrifuged at 13,000 rpm for two minutes, the supernatant was discarded, and the precipitate was suspended in 300 µl of PBS (-). The suspension was centrifuged at 13,000 rpm for two minutes, and the supernatant was discorded. The precipitate was dissolved in 500 µl of 6M guanidine hydrochloride (containing 10 mM Tris-HCl, pH 7.0, and 50 µM CuSO₄) and the solution was sonicated. Next, the solutions was dialyzed against 4 M guanidine hydrochloride (containing 10 mM Tris-HCl, pH 7.0, and 50 µM CuSO₄) for one hour, and hereafter, the dialyzate was again dialyzed against 2 M guanidine hydrochloride (containing 10 mM Tris-HCl, pH 7.0, 50 µM CuSO₄), overnight. The dialyzate was assayed to determine the C-terminal α-amidating activity.

The assay was carried out according to Mizuno et al., B.B.R.C. 137, 984-991, 1986, as follows. Namely, 25 µl of the sample was diluted with water to a total volume of 100 µl, to this solution were added 25 µl of 10 mM N-ethylmaleimide, 25 µl of 10 mM ascorbic acid, 25 µl of 200 µM CuSO₄ , 1.25 µl of 20 mg/ml catalase, 25 µl of 1% rubrol, 2 pmol of [¹²⁵I]-Ac-Tyr-Phe-Gly (170,000 cpm), and 50 µl of 1M Tris-HCl (pH 7.0), and the reaction mixture was incubated at 37°C for 24 hours. After the reaction, to the reaction mixture were added 750 µl of 1M Tris-HCl (pH 7.0) and ethyl acetate, and the whole was mixed and centrifuged. The ethyl acetate layer and aqueous layer were then separated, the radioactivity of both fractions counted by a γ-counter, and a ratio of radioactivity transferred to the ethyl acetate layer was calculated. Note, it has been confirmed that the C-terminal-amidated product, [¹²⁵I]-Ac-Tyr-Phe-NH₂ is selectively transferred to the ethyl acetate layer in the above-method. The result is shown in Fig. 23.

As seen from Fig. 23, W3110/P_{L}chT201 MPF-Sph and W3110/P_{L}chT201 MFK-Sph did not provide the C-terminal amidating activity. Accordingly, it was found that a smaller protein up to the 323th Ala does not provide a sufficient enzyme activity. On the other hand, a larger protein which extends to or beyond the 378th Lys does provide the desired enzyme activity. Furthermore, the position of the N-terminus of the protein derivatives, i.e., the first Met, the 31th Pro or the 37th Pro in the sequence shown in Fig. 1, did not affect the enzyme activity.

Note, the enzyme activity provided by E. coli transformants was relatively low, which is probably due to a lack of glycosylation or an incorrect folding of protein derivative produced by E. coli.

Accordingly, the production of enzyme derivatives by animal cells was attempted as follows.

### Example 10. Construction of animal cell expression plasmids pKDchT201-Sph, pKDchT201-Sal, pKDchT201-S/B, pKDch201-Pst, pKDchT201-Bcl, pKDchT2-Pst and pKDchT2-Bcl

For the expression of enzyme derivatives, plasmids pKDchT201-Sph, pKDchT201-Sal, pKDchT201-S/B, pKDchT201-Pst, and pKDchT201-Bcl (for expression of chT201-type C-terminal α-amidating enzyme derivatives), as well as pKDchT2-Pst and pKDchT2-Bcl (for expression of chT2-type C-terminal α-amidating enzyme derivatives) were constructed from chT201 cDNA, or from chT201 cDNA and chT2 cDNA. These plasmids contained cDNA insert coding for a protein starting from the first amino acid in the amino acid sequence shown in Fig. 1 (or Fig. 3), and terminating at a different amino acid in the amino acid sequence shown in Fig. 1 or that shown in Fig. 3.

These plasmids also contained an SV 40 (Simian Virus 40) early promoter, an intron derived from a rabbit β-globin gene responsible for splicing an RNA and addition of poly A, a cDNA coding for a desired enzyme derivative under the control of the SV40 early promoter, and a poly A addition signal derived from a rabbit β-globin gene, in this order.

Note, E. coli SBM 303 containing the plasmid pKD used for construction of the above-mentioned expression plasmids was deposited with the FRI under the Budapest treaty as FERM BP-2238, on January 11, 1989.

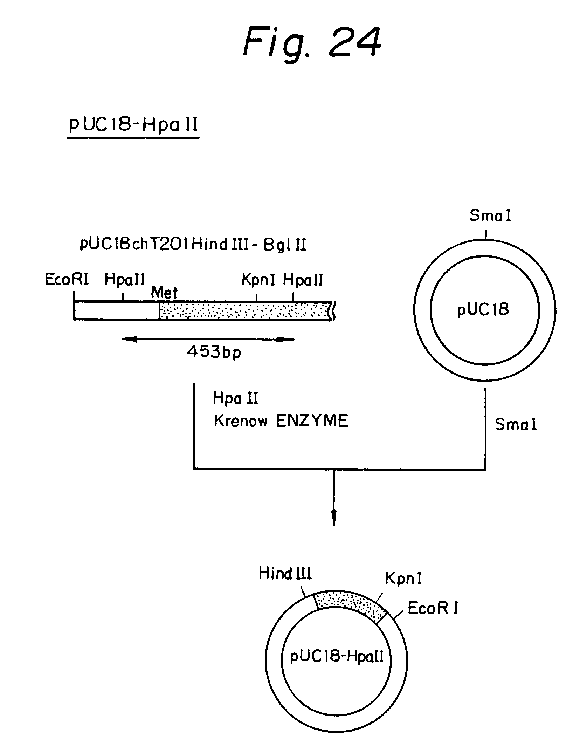

### (1) Construction of pUC18-HpaII (Fig. 24)

As shown in Fig. 24, the plasmid pUC18chT201 HindIII-BglII was cleaved with HpaII to obtain a 453 bp DNA fragment, which was then filled in using Krenow enzyme in the presence of dNTPs to make the ends blunt. The plasmid pUC18 was cleaved with SmaI. The 453 bp DNA and the linearized pUC18 were ligated and used to transform E. coli JM 109, whereby a plasmid pUC18-HpaII was obtained.

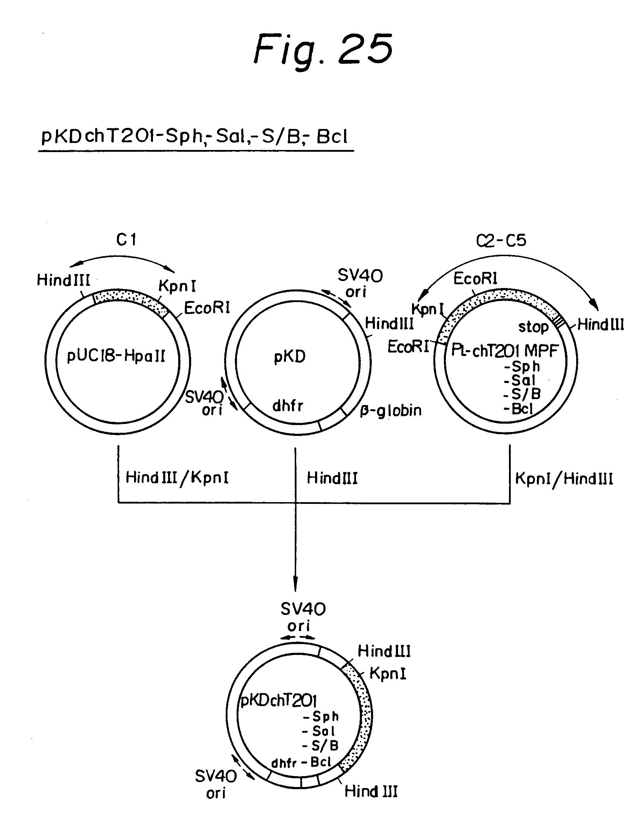

### (2) Construction of plasmids pKDchT201-Sph, pKDchT201-Sal, pKDchT201-S/B and pKDchT201-Bcl (Fig. 25)

The plasmid pUC18 HpaII was cleaved with KpnI and HindIII to obtain a DNA fragment C1, and the P_{L}chT201 MPF-Sph was cleaved with KpnI and HindIII to obtain DNA fragment C2. Further, pKD was cleaved with HindIII. Next the DNA fragments C1 and C2, and the linearized plasmid pKD, were ligated and used to transform E. coli DH1, whereby a plasmid pKDchT201-Sph was obtained.

The same procedure was repeated except that P_{L}chT201 MPF-Sal was used in place of P_{L}chT201 MPF-Sph, to obtain a plasmid PKDchT201-Sal.

Further, the same procedure was repeated except that P_{L}chT201 MPF-S/B was used in place of P_{L}chT201 MPF-Sph, to obtain a plasmid pKDchT201-S/B.

Furthermore, the same procedure was repeated except that P_{L}chT201 MPF-Bcl was used in place of P_{L}chT202 MPF-Sph, to obtain a plasmid pKDchT201-Bcl.

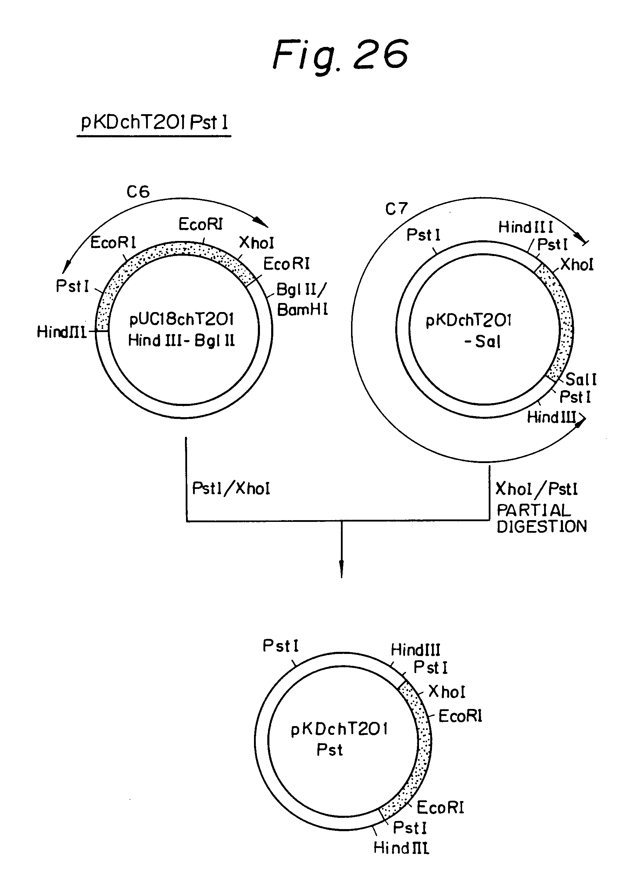

### (3) Construction of pKDchT201-Pst (Fig. 26)

As shown in Fig. 26, the plasmid pUC18chT201 HindIII-BglII was cleaved with PstI and XhoI to obtain a DNA fragment C6. On the other hand, pKDchT201 MPF-Sal was completely digested, with XhoI, and then partially digested with PstI, to obtain a DNA fragment C7. The DNA fragments C6 and C7 were ligated and used to transform E. coli DH1, whereby a plasmid pKDchT201-Pst was obtained.

### (4) Construction of pKDchT2 HindIII-BglII (Fig. 27)

As shown in Fig. 27, the λgt10chT2 phage DNA was cleaved with HindIII and BglII to obtain a DNA fragment containing a chT2 cDNA coding region and a portion of the λgt10 phage. On the other hand, pUC18 was cleaved with HindIII and BamHI. Next, the DNA fragment from the λgt10chT2, and the linearized pUC18, were ligated and used to transform E. coli JM109, whereby a plasmid pUC18chT2 HindIII-BglII was obtained. E. coli transformed with the plasmid pUC18chT2 HindIII-BglII was designated as E. coli SBM304, and deposited with the FRI under the Budapest treaty as FERM BP-2239, on January 11, 1989.

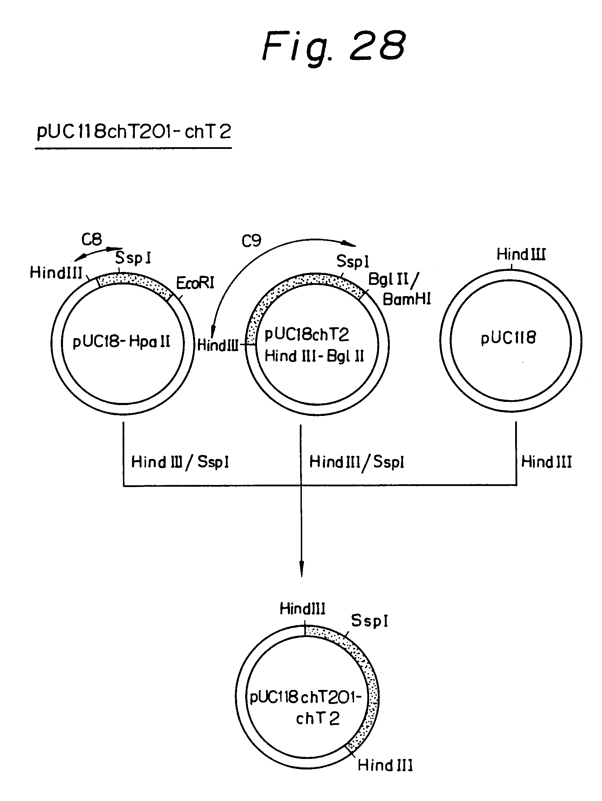

### (5) Construction of pUC18chT201-chT2 (Fig. 28)

As shown in Fig. 28, pUC18 HpaII was cleaved with HindIII and SspI to obtain a DNA fragment C8, and the plasmid pUC18chT2 HindIII-BglII was cleaved with HindIII and SspI to obtain a DNA fragment C9. Further, pUC118 was cleaved with HindIII. Next, the DNA fragments C8 and C9, and the linearized pUC118, were ligated and used to transform E. coli JM109, whereby a plasmid pUC118chT201-chT2 was obtained.

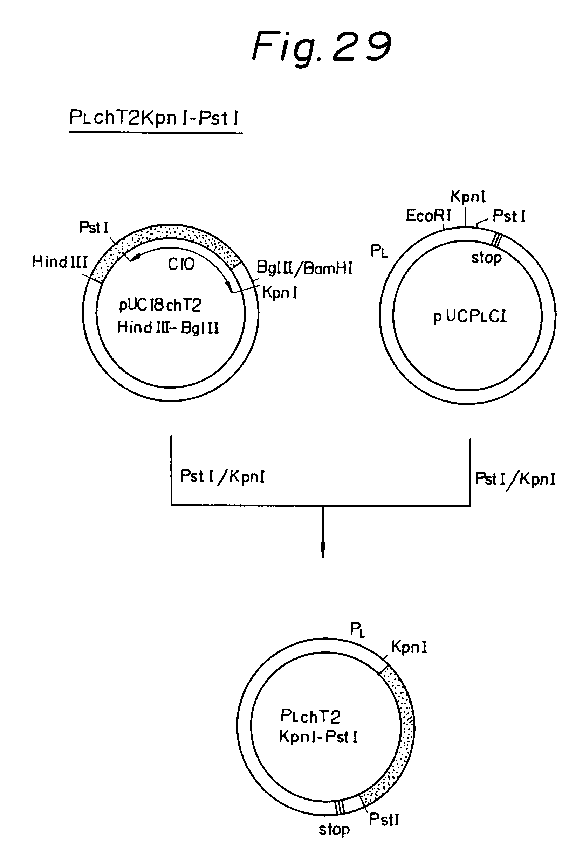

### (6) Construction of P_{L}chT2 KpnI-PstI (Fig. 29)

As shown in Fig. 29, the plasmid pUC18chT2 HindIII-BglII was cleaved with KpnI and PstI to obtain a DNA fragment c10, and the plasmid pUCP_{L}CI was cleaved with KpnI. Next, the DNA fragment C10 and the linearized pUCP_{L}CI were ligated and used to transform E. coli JM109, whereby a plasmid P_{L}chT2 KpnI-PstI was obtained.

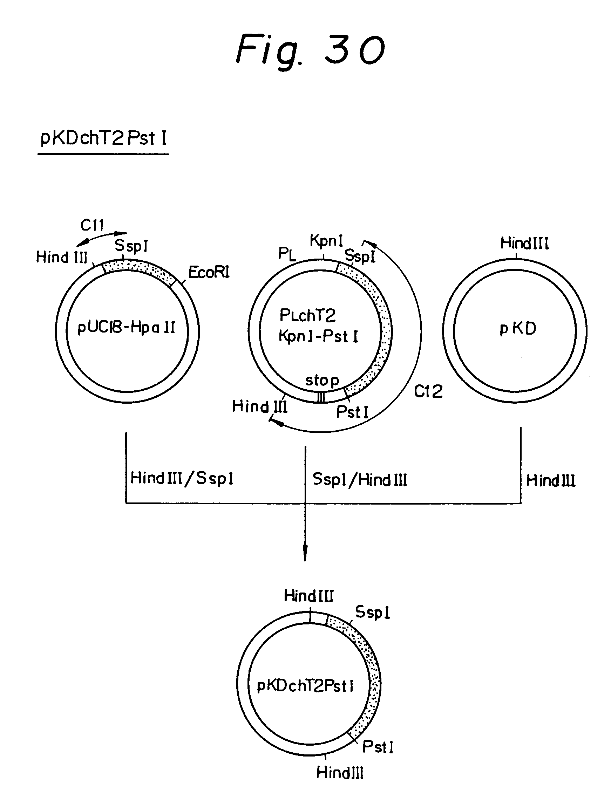

### (7) Construction of pKDchT2-Pst (Fig. 30)

As shown in Fig. 30, the plasmid pUC18 HpaII was cleaved with HindIII and SsPI to obtain a DNA fragment C11, and the plasmid P_{L}chT2 KpnI-PstI was cleaved with SspI and HindIII to obtain a DNA fragment C12. Further, the plasmid pKD was cleaved with HindIII. Next, the DNA fragments C11 and C12, and the linearized pKD, were ligated and used to transform E. coli DH1, whereby a plasmid pKDchT2-Pst was obtained.

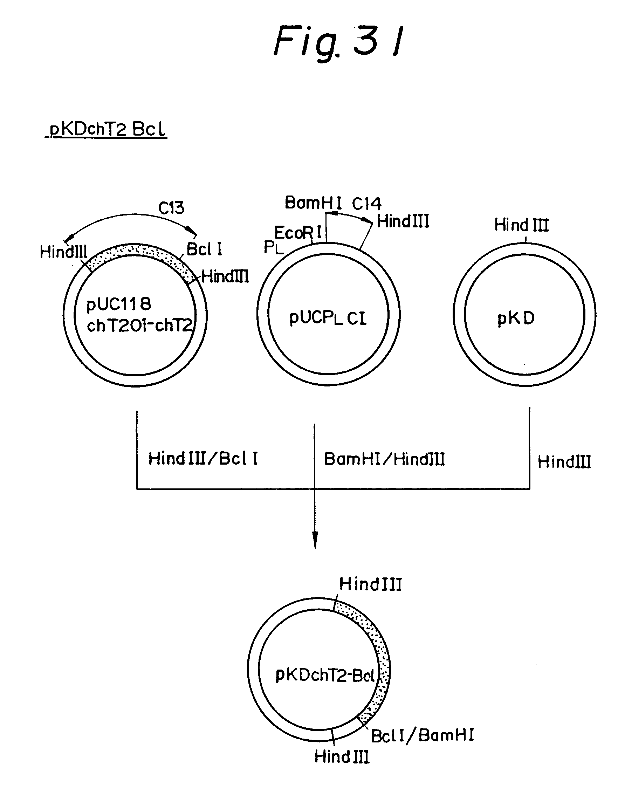

### (8) Construction of pKDchT2-Bcl (Fig. 31)

As shown in Fig. 31, the plasmid pUC118chT201-chT2 was cleaved with HindIII and BclI to obtain a DNA fragment C13, and the plasmid pUCP_{L}CI was cleaved with BamHI and HindIII to obtain a DNA fragment C14. Further, the plasmid pKD was cleaved with HindIII. Next, the DNA fragments C13 and C14, and the linearized pKD, were ligated and used to transform E. coli DH1, whereby a plasmid pKDchT2-Bcl was obtained.

According to the above-mentioned construction processes, five expression plasmids, pKDchT201-Sph, pKDchT201-Sal, pKDchT201-S/B, pKDchT201-Pst, and pKDchT201-Bcl derived from chT201 cDNA and coding for chT201-type protein derivatives, as well as two expression plasmids, pKDchT2-Pst and pKDchT2-Bcl derived from chT2 cDNA and chT201 cDNA and coding for chT101-type protein derivatives, were constructed.

### Example 11. Expression of protein derivatives in COSl cells

The Expression of C-terminal α-amidating enzymes of human thyroid origin was tested as follows.

### (1) Transfection

Plasmids pKD, pKDchT201-Sph, pKDchT201-Sal, pKDchT201-S/B, pKDchT201-Bcl, pKDchT201-Pst, pKDchT2-Pst and pKDchT2-Bcl were prepared from corresponding E. coli transformants by a conventional procedure, 30 µg of each plasmid DNA was precipitated by ethanol, and the ethanol precipitate was washed with 80% ethanol and dried under vacuum. The dried DNA was dissolved in 720 µl of sterile distilled water, and to this solution was added 720 µl of an A buffer of a Cell Phect transfection kit (Pharmacia), and the whole was thoroughly mixed and allowed to stand at a room temperature for 10 minutes. Next, 1440 µl of B buffer of the same kit was added, and the whole was thoroughly mixed and allowed to stand at a room temperature for 15 minutes, whereby a calcium phosphate-DNA complex was formed.

On the other hand, COSl cells were cultured overnight in 30 medium at a concentration of 7.5 x 10⁵ cell/10 cm petri dish, and after adding 10 ml of fresh medium per petri dish, further cultured for 4 hours. To the culture was added 960 µl/petri dish of the calcium phosphate-DNA complex, the culture was incubated for 4 hours, the supernatant was then removed by suction, and the petri dish was washed with 5 ml of a fetal calf serum-free medium. Next, 2.5 ml of a medium containing 20% glycerol was added to the dish and was allowed to stand for one minute. After removing the glycerol-containing medium, the petri dish was washed with 8 ml of a medium, 10 ml of the medium was added to the petri dish, and culturing was carried out for 3 days. Usually, the medium was Dulbecco's MEM containing 10% fetal calf serum (Flow Lab.), and culturing was carried out at 37°C in an atmosphere of 50% CO₂.

### Example 12. Determination of C-terminal α-amidating activity of culture

After the three day culture of transfected COSl cells as described above, the supernatant was obtained and centrifuged at 15000 rpm for 4 minutes to obtain a supernatant, and the C-terminal α-amidating activity of the supernatant was determined by an assay. The assay was carried out according to Mizuno et al. (supra), and the conversion of [¹²⁵I]-Ac-Tyr-Phe-Gly to [¹²⁵I]-Ac-Tyr-Phe-NH₄ was measured. The result is shown in Figs. 32(A) and 32(B). This result was obtained by reacting 100 µl of the supernatant for 16 hours.

As seen from Figs. 32(A) and 32 (B), (1) both proteins encoded by chT201 DNA and chT2 cDNA respectively have a C-terminal α-amidating enzyme activity; and
(2) The activity is provided not only by a protein having a full length primary amino acid sequence encoded by chT201 cDNA or chT2 cDNA, but also by a shortened protein having a portion of an amino acid sequence encloded by the chT201 cDNA or chT2 cDNA.

In practice, the expression vector such as pKDchT201-Bcl, pKDchT2-Bcl, pKDchT201-Pst, pKDcht2-Pst, pKDchT201-S/B, and pKDchT201-Sal coding for a C-terminal truncated protein derivative, when introduced to COSl cells, exhibited a significant C-terminal α-amidating enzyme activity in a culture supernatant.

The pKDchT201-Sph, however, which encodes an amino acid sequence from the first Met to 323th Ala in the amino acid sequence shown in Fig. 1, did not exhibit the desired enzyme activity. Accordingly, it was found that a protein having an amino acid sequence which terminates at or before the 323th Ala, is insufficient, but a protein having an amino acid sequence which extends to or beyond the 378th Lys has a size sufficient to exhibit the C-terminal α=amidating enzyme activity.

According to the present invention, a large amount of C-terminal α-amidating enzyme can be produced using cloned cDNA derived from human thyroid origin. Accordingly, C-terminal amidated peptides or proteins, which are useful and important in the pharmaceutical or medical field and other industrial fields, can be economically produced.

Reference under Rule 13-2 of the Budapest treaty to deposited microorganisms:
Depository Institute: Fermentation Research Institute, Agency of Industrial Science and Technology;
Address: 1-3, Tsukuba-shi 1-chome, Ibaraki-ken, 301 Japan;
Identification of deposited microorganisms, deposition numbers and deposition date;
1. E. coli DH1/PXA799 FERM BP-1586 December 3, 1987.
2. E. coli SBM302 FERM BP-2237 January 11, 1989.
3. E. coli SBM303 FERM BP-2238 January 11, 1989.
4. E. coli SBM304 FERM BP-2239 January 11, 1989.
5. E. coli SBM305 FERM BP-2240 January 11, 1989.

## Claims (Claims for the following Contracting State(s): AT, BE, CH, LI, DE, DK, FR, GB, IT, LU, NL, SE)

1. A C-terminal α-amidating enzyme which comprises an amino acid sequence according to that shown in Fig. 1 from position 37 (Pro) to position 378 (Lys), or a derivative of that sequence maintaining the enzyme activity.

2. A C-terminal α-amidating enzyme according to claim 1, having an amino acid sequence according to that shown in Fig. 1, starting at an amino acid from position 1 (Met) to position 37 (Pro) inclusive and terminating at an amino acid from position 378 (Lys) to position 974 (Ser) inclusive, or a derivative of that sequence maintaining the enzyme activity.

3. A C-terminal α-amidating enzyme according to claim 1, having an amino acid sequence according to that shown in Fig. 1, starting at an amino acid from position 1 (Met) to position 37 (Pro) inclusive, continuing until an amino acid from position 378 (Lys) to position 974 (Ser), and having there an additional C-terminal sequence of from one to ten amino acids.

4. A C-terminal α-amidating enzyme which comprises an amino acid sequence according to that shown in Fig. 3 from position 37 (Pro) to position 378 (Lys), or a derivative of that sequence maintaining the enzyme activity.

5. A C-terminal α-amidating enzyme according to claim 4, having an amino acid sequence according to that shown in Fig. 3, starting at an amino acid from position 1 (Met) to position 37 (Pro) inclusive and terminating at an amino acid from position 378 (Lys) to position 866 (Ser) inclusive, or a derivative of that sequence maintaining the enzyme activity.

6. A C-terminal α-amidating enzyme according to claim 4, having an amino acid sequence according to that shown in Fig. 3, starting at an amino acid from position 1 (Met) to position 37 (Pro) inclusive, continuing until an amino acid from position 378 (Lys) to position 866 (Ser), and having there an additional C-terminal sequence of from one to ten amino acids.

7. A process for production of C-terminal α-amidating enzyme according to any one of the preceding claims, comprising
(a) cultivating prokaryotic or eukaryotic cells transformed with an expression vector containing a gene coding for the enzyme, to produce the enzyme, and
(b) recovering the enzyme from the culture.

8. A gene coding for a C-terminal α-amidating enzyme according to any one of claims 1 to 6.

9. A plasmid incorporating a gene according to claim 8.

10. Prokaryotic or eukaryotic cells transfected with a plasmid according to claim 9.

## Claims (Claims for the following Contracting State(s): ES)

1. A process for production of a C-terminal α-amidating enzyme, the said enzyme comprising an amino acid sequence according to that shown in Fig. 1 from position 37 (Pro) to position 378 (Lys), or a derivative of that sequence maintaining the enzyme activity, the said process comprising
(a) cultivating prokaryotic or eukaryotic cells transformed with an expression vector containing a gene coding for the enzyme, to produce the enzyme, and
(b) recovering the enzyme from the culture.

2. A process according to claim 1 in which the said C-terminal α-amidating enzyme has an amino acid sequence according to that shown in Fig. 1, starting at an amino acid from position 1 (Met) to position 37 (Pro) inclusive and terminating at an amino acid from position 378 (Lys) to position 974 (Ser) inclusive, or a derivative of that sequence maintaining the enzyme activity.

3. A process according to claim 1 in which the said C-terminal α-amidating enzyme has an amino acid sequence according to that shown in Fig. 1, starting at an amino acid from position 1 (Met) to position 37 (Pro) inclusive, continuing until an amino acid from position 378 (Lys) to position 974 (Ser), and has there an additional C-terminal sequence of from one to ten amino acids.

4. A process for production of a C-terminal α-amidating enzyme, the said enzyme comprising an amino acid sequence according to that shown in Fig. 3 from position 37 (Pro) to position 378 (Lys), or a derivative of that sequence maintaining the enzyme activity, the said process comprising
(a) cultivating prokaryotic or eukaryotic cells transformed with an expression vector containing a gene coding for the enzyme, to produce the enzyme, and
(b) recovering the enzyme form the culture.

5. A process according to claim 4 in which the said C-terminal α-amidating enzyme has an amino acid sequence according to that shown in Fig. 3, starting at an amino acid from position 1 (Met) to position 37 (Pro) inclusive and terminating at an amino acid from position 378 (Lys) to position 866 (Ser) inclusive, or a derivative of that sequence maintaining the enzyme activity.

6. A process according to claim 4 in which the said C-terminal α-amidating enzyme has an amino acid sequence according to that shown in Fig. 3, starting at an amino acid from position 1 (Met) to position 37 (Pro) inclusive, continuing until an amino acid from position 378 (Lys) to position 866 (Ser), and has there an additional C-terminal sequence of from one to ten amino acids.

7. Prokaryotic or eukaryotic cells transfected with a plasmid incorporating a gene coding for a C-terminal α-amidating enzyme produced according to any of the processes described in any one of claims 1 to 6.

## Patentansprüche (Patentansprüche für folgende(n) Vertragsstaat(en): AT, BE, CH, LI, DE, DK, FR, GB, IT, LU, NL, SE)

1. C-terminal α-amidierendes Enzym, das eine Aminosäuresequenz nach der in Fig. 1 gezeigten von der Position 37 (Pro) bis zur Position 378 (Lys) umfaßt, oder Derivat dieser Sequenz, das die Enzymaktivität beibehält.

2. C-terminal α-amidierendes Enzym nach Anspruch 1 mit einer Aminosäuresequenz nach der in Fig. 1 gezeigten, die bei einer Aminosäuresequenz von der Position 1 (Met) bis einschließlich zur Position 37 (Pro) beginnt und bei einer Aminosäure von der Position 378 (Lys) bis einschließlich zur Position 974 (Ser) endet, oder ein Derivat dieser Sequenz, das die Enzymaktivität beibehält.

3. C-terminal α-amidierendes Enzym nach Anspruch 1 mit einer Aminosäuresequenz nach der in Fig. 1 gezeigten, die bei einer Aminosäure von der Position 1 (Met) bis einschließlich zur Position 37 (Pro) beginnt, sich bis zu einer Aminosäure der Position 378 (Lys) zur Position 974 (Ser) fortsetzt, und eine zusätzliche C-terminale Sequenz aus 1 bis 10 Aminosäuren aufweist.

4. C-terminal α-amidierendes Enzym, das eine Aminosäuresequenz nach der in Fig. 3 gezeigten von der Position 37 (Pro) bis zur Position 378 (Lys) umfaßt, oder ein Derivat dieser Sequenz, das die Enzymaktivität beibehält.

5. C-terminal α-amidierendes Enzym nach Anspruch 4 mit einer Aminosäuresequenz nach der in Fig. 3 gezeigten, die bei einer Aminosäure von der Position 1 (Met) bis einschließlich zur Position 37 (Pro) beginnt und bei einer Aminosäure von der Position 378 (Lys) bis einschließlich zur Position 866 (Ser) endet, oder ein Derivat mit dieser Sequenz, das die Enzymaktivität beibehält.

6. C-terminal α-amidierendes Enzym nach Anspruch 4 mit einer Aminosäuresequenz nach der in Fig. 3 gezeigten, die bei einer Aminosäure von der Position 1 (Met) bis einschließlich zur Position 37 (Pro) beginnt, sich bis zu einer Aminosäure von der Position 378 (Lys) bis zur Position 866 (Ser) fortsetzt und eine zusätzliche C-terminale Sequenz aus 1 bis 10 Aminosäuren aufweist.

7. Verfahren zur Herstellung eines den C-Terminus α-amidierenden Enzyms nach einem der vorstehenden Ansprüche, welches umfaßt:
(a) Züchten prokaryotischer oder eukaryotischer Zellen, die mit einem Expressionsvektor transformiert sind, der ein Gen enthält, das dieses Enzym codiert, wodurch das Enzym produziert wird, und
(b) Gewinnung des Enzyms aus der Kultur.

8. Gen, das das den C-Terminus α-amidierende Enzym nach einem der Ansprüche 1 bis 6 codiert.

9. Plasmid, das ein Gen nach Anspruch 8 einführt.

10. Prokaryotische oder eukaryotische Zellen, die durch Transformation mit einem Plasmid nach Anspruch 9 infiziert sind.

## Patentansprüche (Patentansprüche für folgende(n) Vertragsstaat(en): ES)

1. Verfahren zur Herstellung eines den C-Terminus α-amidierenden Enzyms, wobei das Enzym eine Aminosäuresequenz nach der in Fig. 1 gezeigten von der Position 37 (Pro) bis zur Position 378 (Lys) umfaßt, oder eines Derivats mit dieser Sequenz, das die Enzymaktivität beibehält, wobei das Verfahren umfaßt:
(a) Züchten prokaryotischer oder eukaryotischer Zellen, die mit einem Expressionsvektor transformiert sind, der ein Gen enthält, das dieses Enzym codiert, wodurch das Enzym produziert wird, und
(b) Gewinnung des Enzyms aus der Kultur.

2. Verfahren nach Anspruch 1, wobei das C-terminal α-amidierende Enzym eine Aminosäuresequenz nach der in Fig. 1 gezeigten aufweist, die bei einer Aminosäure von der Position 1 (Met) bis einschließlich zur Position 37 (Pro) beginnt und bei einer Aminosäure von der Position 378 (Lys) bis einschließlich zur Position 974 (Ser) endet, oder eines Derivats dieser Sequenz, das die Enzymaktivität beibehält.

3. Verfahren nach Anspruch 1, wobei das C-terminal α-amidierende Enzym eine Aminosäuresequenz nach der in Fig. 1 gezeigten aufweist, die bei einer Aminosäure von der Position 1 (Met) bis einschließlich zur Position 37 (Pro) beginnt, sich bis zu einer Aminosäure von der Position 378 (Lys) bis zur Position 974 (Ser) fortsetzt, und eine zusätzliche C-terminale Sequenz aus 1 bis 10 Aminosäuren aufweist.

4. Verfahren zur Herstellung eines C-terminal α-amidierenden Enzyms, wobei das Enzym eine Aminosäuresequenz nach der in Fig. 3 gezeigten von der Position 37 (Pro) bis zur Position 378 (Lys) umfaßt, oder eines Derivats dieser Sequenz, das die Enzymaktivität beibehält, wobei das Verfahren umfaßt:
(a) Züchten prokaryotischer oder eukaryotischer Zellen, die mit einem Expressionsvektor transformiert sind, der ein Gen enthält, das dieses Enzym codiert, wodurch das Enzym produziert wird, und
(b) Gewinnung des Enzyms aus der Kultur.

5. Verfahren nach Anspruch 4, wobei das C-terminal α-amidierende Enzym eine Aminosäuresequenz nach der in Fig. 3 gezeigten aufweist, die bei einer Aminosäure von der Position 1 (Met) bis einschließlich zur Position 37 (Pro) beginnt und bei einer Aminosäure von der Position 378 (Lys) bis einschließlich zur Position 866 (Ser) endet, oder eines Derivats dieser Sequenz, das die Enzymaktivität beibehält.

6. Verfahren nach Anspruch 4, wobei das C-terminal α-amidierende Enzym eine Aminosäuresequenz nach der in Fig. 3 gezeigten aufweist, die bei einer Aminosäure von der Position 1 (Met) bis einschließlich zur Position 37 (Pro) beginnt, sich bis zu einer Aminosäure von der Position 378 (Lys) bis zur Position 866 (Ser) fortsetzt und eine zusätzliche C-terminale Sequenz aus 1 bis 10 Aminosäuren aufweist.

7. Prokaryotische oder eukaryotische Zellen, die durch Transformation mit einem Plasmid infiziert sind, das ein Gen einführt, das ein C-terminal α-amidierendes Enzyms codiert, das nach einem der Verfahren hergesellt wurde, die in einem der Ansprüche 1 bis 6 beschrieben sind.

## Revendications (Revendications pour l'(les) Etat(s) contractant(s) suivant(s): AT, BE, CH, LI, DE, DK, FR, GB, IT, LU, NL, SE)

1. Une enzyme d'α-amidification en position C-terminale qui comprend une séquence d'acides aminés telle que représentée à la figure 1, allant de la position 37 (Pro) à la position 378 (Lys), ou un dérivé de cette séquence conservant l'activité enzymatique.

2. Une enzyme d'α-amidification en position C-terminale selon la revendication 1, présentant une séquence d'acides aminés telle que représentée dans la figure 1, commençant au niveau de l'acide aminé compris entre la position 1 (Met) et la position 37 (Pro) bornes incluses, et se terminant à un acide aminé compris entre la position 378 (Lys) et la position 974 (Ser) bornes incluses, ou un dérivé de cette séquence conservant l'activité enzymatique.

3. Une enzyme d'α-amidification en position C-terminale selon la revendication 1, présentant une séquence d'acides aminés telle que représentée dans la figure 1, commençant au niveau d'un acide aminé compris entre la position 1 (Met) et la position 37 (Pro) bornes incluses, et se poursuivant jusqu'à un acide aminé compris entre la position 378 (Lys) et la position 974 (Ser) et comportant à ce niveau une séquence C-terminale additionnelle comportant de 1 à 10 acides aminés.

4. Une enzyme d'α-amidification en position C-terminale qui comprend une séquence d'acides aminés telle que représentée dans la figure 3, allant de la position 37 (Pro) à la position 378 (Lys), ou un dérivé de cette séquence conservant l'activité enzymatique.

5. Une enzyme d'α-amidification en position C-terminale selon la revendication 4, présentant une séquence d'acides aminés telle que représentée dans la figure 3, commençant au niveau d'un acide aminé compris entre la position 1 (Met) et la position 37 (Pro) bornes incluses, et se terminant au niveau d'un acide aminé compris entre la position 378 (Lys) et la position 866 (Ser) bornes incluses, ou un dérivé de cette séquence concernant l'activité enzymatique.

6. Une enzyme d'α-amidification en position C-terminale selon la revendication 4, présentant une séquence d'acides aminés telle que représentée dans la figure 3, commençant au niveau d'un acide aminé compris entre la position 1 (Met) à la position 37 (Pro) bornes incluses, et se poursuivant jusqu'à un acide aminé compris entre la position 378 (Lys) et la position 866 (Ser), et comportant à ce niveau une séquence C-terminale supplémentaire comprenant 1 à 10 acides aminés.

7. Un procédé de production d'une enzyme d'α-amidification en position C-terminale selon l'une quelconque des revendications précédentes, comprenant:
(a) la culture des cellules procaryotes ou eucaryotes transformées avec un vecteur d'expression contenant un gène codant pour l'enzyme, pour produire l'enzyme; et
(b) récupération de l'enzyme à partir de la culture.

8. Un gène codant pour une enzyme d'α-amidification en position C-terminale selon l'une quelconque des revendications 1 à 6.

9. Un plasmide incorporant un gène selon la revendication 8.

10. Cellules procaryotes ou eucaryotes transfectées par un plasmide selon la revendication 9.

## Revendications (Revendications pour l'(les) Etat(s) contractant(s) suivant(s): ES)

1. Un procédé de production d'une enzyme d'α-amidification en position C-terminale, ladite enzyme comprenant une séquence d'acides aminés telle que représentée dans la figure 1, allant de la position 37 (Pro) à la position 378 (Lys), ou un dérivé de cette séquence conservant l'activité enzymatique, ledit procédé comprenant:
(a) la culture des cellules procaryotes ou eucaryotes transformées avec un vecteur d'expression contenant un gène codant pour l'enzyme, pour produire l'enzyme; et
(b) récupération de l'enzyme à partir de la culture.

2. Un procédé selon la revendication 1, dans lequel ladite enzyme d'α-amidification en position C-terminale présente une séquence d'acides aminés telle que représentée dans la figure 1, commençant au niveau de l'acide aminé compris entre la position 1 (Met) et la position 37 (Pro) bornes incluses, et se terminant à un acide aminé compris entre la position 378 (Lys) et la position 974 (Ser) bornes incluses, ou un dérivé de cette séquence conservant l'activité enzymatique.

3. Un procédé selon la revendication 1, dans lequel ladite enzyme d'α-amidification en position C-terminale présente une séquence d'acides aminés telle que représentée dans la figure 1, commençant au niveau d'un acide aminé compris entre la position 1 (Met) et la position 37 (Pro) bornes incluses, et se poursuivant jusqu'à un acide aminé compris entre la position 378 (Lys) et la position 974 (Ser) et comportant à ce niveau une séquence C-terminale additionnelle comportant de 1 à 10 acides aminés.

4. Un procédé de production d'une enzyme d'α-amidification en position C-terminale, ladite enzyme comprenant une séquence d'acides aminés telle que représentée dans la figure 3, allant de la position 37 (Pro) à la position 378 (Lys), ou un dérivé de cette séquence conservant l'activité enzymatique, ledit procédé comprenant:
(a) la culture des cellules procaryotes ou eucaryotes transformées avec un vecteur d'expression contenant un gène codant pour l'enzyme, pour produire l'enzyme; et
(b) récupération de l'enzyme à partir de la culture.

5. Un procédé selon la revendication 4, dans lequel ladite enzyme d'α-amidification en position C-terminale présente une séquence d'acides aminés telle que représentée dans la figure 3, commençant au niveau d'un acide aminé compris entre la position 1 (Met) et la position 37 (Pro) bornes incluses, et se terminant au niveau d'un acide aminé compris entre la position 378 (Lys) et la position 866 (Ser) bornes incluses, ou un dérivé de cette séquence concernant l'activité enzymatique.

6. Un procédé selon la revendication 4, dans lequel ladite enzyme d'α-amidification en position C-terminale présente une séquence d'acides aminés telle que représentée dans la figure 3, commençant au niveau d'un acide aminé compris entre la position 1 (Met) à la position 37 (Pro) bornes incluses, et se poursuivant jusqu'à un acide aminé compris entre la position 378 (Lys) et la position 866 (Ser), et comportant à ce niveau une séquence C-terminale supplémentaire comprenant 1 à 10 acides aminés.

7. Cellules procaryotes ou eucaryotes transfectées par un plasmide incorporant un gène codant pour une enzyme d'α-amidification en position C-terminale produite selon l'un quelconque des procédés décrits dans l'une quelconque des revendications 1 à 6.
